# EUROPEAN PATENT APPLICATION

(11) **EP 3 628 681 A2**
(43) Date of publication of application: **01.04.2020**
(21) Application number: 18794624.9
(22) Date of filing: 05.05.2018
(51) Int. Cl.: C07K 14/485, C07K 14/49, C07K 14/535, C07K 14/515, C07K 14/59, A61K 38/00

(54) **METHOD FOR EXTENDING HALF-LIFE OF PROTEIN**

(30) Priority: 05.05.2017 WO PCT/KR2017/004730
(71) Applicant: Ubiprotein, Corp., Seongnam-si, Gyeonggi-do 13493 (KR)
(72) Inventor: BAEK, Kwang-Hyun, Seoul 06291 (KR); KIM, Kyunggon, Seoul 05507 (KR); KIM, Myung-Sun, Wonju-si Gangwon-do 26438 (KR); KIM, Hyeonmi, Suwon-si Gyeonggi-do 16687 (KR); OH, Su Kyung, Yongin-si Gyeonggi-do 16823 (KR); LEE, Mi Jeong, Seoul 02256 (KR); BAE, Sung-ryul, Seongnam-si Gyeonggi-do 13602 (KR)
(74) Representative: Bringer IP
(86) International application number: PCT/KR2018/005239
(87) International publication number: WO 2018/203737

(57) **Abstract**

The present invention relates to a method for prolonging the half-life of a protein, the method comprising substituting at least one lysine residue present in the amino acid sequence of the protein, and to a protein having an extended half-life. The protein of the present invention, in which the lysine residue has been substituted, remains in the human body for a long time and exhibits an excellent therapeutic effect. The protein of the present invention includes EGF, PDGFA and PDGFB, GM-CSF, FSH-alpha and FSH-beta, and ANGPT-1.

## Description

### Technical Field

The present invention relates to a method for prolonging the half-life of a protein or (poly)peptide by substitution of at least one amino acid residue in the protein or (poly)peptide. In addition, the present invention relates to a protein or (poly)peptide with an extended half-life, produced by such a method.

### Background Art

Intracellular proteolysis occurs through two pathways, that is, lysosome pathway and proteasome pathway. The lysosome pathway, by which 10% to 20% of proteins is degraded, lacks substrate specificity and delicate temporal regulation characteristics. In other words, the lysosome pathway is mostly a process by which extracellular or membrane proteins are degraded as if cell surface proteins that have been incorporated into cells by endocytosis are degraded in lysosome. However, in order for proteins to be selectively degraded in eukaryotic cells, the proteins have to undergo ubiquitin-proteasome pathway (UPP), that is, a process in which ubiquitin binds to a target protein by ubiquitin-conjugating enzyme so that a polyubiquitin chain is formed, and such a polyubiquitin chain is recognized and degraded by proteasome. 80% to 90% or more of eukaryotic proteins are degraded through this process. The ubiquitin-proteasome pathway regulates most proteolysis occurring in eukaryotic cells, and thus is responsible for protein conversion and homeostasis.

Ubiquitin is a protein composed of 76 amino acids which are highly conserved, and is present in almost all eukaryotic cells. Among the amino acids thereof, the 6^{th}, 11^{th}, 27^{th}, 29^{th}, 33^{rd}, 48^{th}, and 63^{rd} amino acid residues are lysine (Lys, K) residues, and the 48^{th} and 63^{rd} lysine residues play a major role in formation of a polyubiquitin chain. A process (ubiquitination) by which a protein is labeled with ubiquitin involves a series of enzyme systems (E1, E2, E3), and the labeled protein is degraded by the 26S proteasome, an ATP-dependent protease complex. The ubiquitin-proteasome pathway involves two distinct, consecutive processes. The first process is a process by which a substrate protein is covalently labeled with multiple ubiquitin molecules, and the second process is a process by which the ubiquitin-labeled protein is degraded by the complex 26S proteasome. Binding between ubiquitin and a substrate protein occurs through an isopeptide bond between a lysine residue of the substrate protein molecule and the C-terminal glycine of ubiquitin, and ubiquitination is achieved by ubiquitin-activating enzyme (E1), ubiquitin-conjugating enzyme (E2), and ubiquitin protein ligase (E3) which form thiol esters with ubiquitin. Among these, the ubiquitin-activating enzyme (E1) activates ubiquitin in an ATP-dependent response. The ubiquitin-conjugating enzyme (E2) receives the activated ubiquitin from E1 at a cysteine residue in its ubiquitin-conjugated domain, and delivers the activated ubiquitin to the E3 ligase or directly to the substrate protein. The E3 enzyme also catalyzes a stable isopeptide bond between the lysine residue of the substrate protein and the glycine residue of the ubiquitin. Another ubiquitin may be linked to the C-terminal lysine residue of the ubiquitin bound to the substrate protein. Repetition of this process causes several ubiquitin molecules to be linked to the substrate protein in a branched shape so that a polyubiquitin chain is formed. In a case where the polyubiquitin chain is formed, the protein is recognized and selectively degraded by the 26S proteasome.

Meanwhile, various types of proteins and (poly)peptides, which have a therapeutic effect *in vivo,* are known. Examples of such proteins or (poly)peptides, which have a therapeutic effect *in vivo,* may include growth hormone releasing hormone (GHRH), growth hormone releasing peptide, interferons (interferon-a or interferon-β), interferon receptors, colony stimulating factors (CSFs), glucagon-like peptides, interleukins, interleukin receptors, enzymes, interleukin binding proteins, cytokine binding proteins, G-protein-coupled receptors, human growth hormone (hGH), macrophage activating factor, macrophage peptide, B cell factor, T cell factor, protein A, allergy inhibitors, cell necrosis glycoproteins, G-protein-coupled receptors, immunotoxins, lymphotoxins, tumor necrosis factor, tumor suppressors, metastasis growth factor, alpha-1 antitrypsin, albumin, alpha-lactalbumin, apolipoprotein-E, erythropoietin, highly glycosylated erythropoietin, angiopoietins, hemoglobin, thrombin, thrombin receptor activating peptide, thrombomodulin, factor VII, factor VIIa, factor VIII, factor IX, factor XIII, plasminogen activating factor, urokinase, streptokinase, hirudin, protein C, C-reactive protein, renin inhibitors, collagenase inhibitors, superoxide dismutase, leptin, platelet-derived growth factor, epithelial growth factor, epidermal growth factor, angiostatin, angiotensin, bone growth factor, bone stimulating protein, calcitonin, insulin, atriopeptin, cartilage inducing factor, fibrin-binding peptide, elcatonin, connective tissue activating factor, tissue factor pathway inhibitors, follicle stimulating hormone, luteinizing hormone, luteinizing hormone releasing hormone, nerve growth factors, parathyroid hormone, relaxin, secretin, somatomedin, insulin-like growth factor, adrenocortical hormone, glucagon, cholecystokinin, pancreatic polypeptide, gastrin releasing peptide, corticotropin releasing factor, thyroid stimulating hormone, autotaxin, lactoferrin, myostatin, receptors, receptor antagonists, cell surface antigens, virus derived vaccine antigens, monoclonal antibodies, polyclonal antibodies, and antibody fragments.

Epidermal growth factor (EGF) binds to its receptor, EGFR, so that cell growth, proliferation, and differentiation are promoted; and human EGF consists of 53 amino acids (Exp Cell Res., 284 (1): 2-13, 2003). In addition, it is reported that such EGF plays an important role in skin and hair regeneration (J Dermatol Sci., 72 (2): 81-86, 2013).

Platelet-derived growth factor (PDGF), which is one of growth factors, regulates cell growth and division, participates in angiogenesis, and exists as a heterodimer in which platelet-derived growth factor subunit A (PDGFA) and platelet-derived growth factor subunit B (PDGFB) have respective functions (Biochim. Biophys. Acta., 989 (1): 110, 1989; EMBO J., 11 (12): 4251-4259, 1992). PDGF is synthesized and stored in platelet alpha granules. PDGF is secreted when platelet becomes active, and is also produced in cells such as muscle cells, activated macrophages, and epidermal cells (Curr Pharm Des., 19 (19): 3384-3390,2013).

Granulocyte-macrophage colony-stimulating factor (GM-CSF), a protein secreted by macrophages, T cells, mast cells, natural killer cells, endothelial cells, and fibroblasts, is a cytokine that functions as a leukocyte growth factor. In addition, GM-CSF stimulates stem cells to produce granulocytes (neutrophils, basophils, and eosinophils) and monocytes, and rapidly increases the number of macrophages to fight infection, which allows GM-CSF to act on immune/infection reactions (Med Oncol., 31 (1): 774, 2014; Blood, 77 (6): 1131-1145, 1991). It has been reported that recombinant GM-CSF can be used as a vaccine adjuvant in HIV-infected patients (Hum Vaccine Immunother., 8 (11): 1654-1658, 2012; Vaccines (Basel)., 2 (1): 160178, 2014). In addition, it is reported that GM-CSF is effective in increasing leukocyte counts in a case where patients with non-Hodgkin lymphoma, leukemia/lymphoma, or Hodgkin lymphoma are subjected to bone marrow transplantation, while, from the viewpoint that GM-CSF is highly expressed in the joints of patients with rheumatoid arthritis, decreased level of GM-CSF can decrease inflammation or damage (Expert Rev Neurother., 13 (3): 313-335,2013).

Follicle-stimulating hormone (FSH) is a gonadotrophin in the form of a 35.5 kDa glycoprotein heterodimer that consists of two polypeptide units, alpha and beta. FSH, which plays a role in promoting and maintaining follicular growth in women and sperm development in men, is synthesized in and secreted by gonadotropic cells of the anterior pituitary gland, and regulates reproductive processes, development, growth, and pubertal maturation of the body (Annu Rev Biochem., 50: 465-495, 1981; Proc Natl Acad Sci USA, 109 (31): 12491-12496, 2012). In general, follicle-stimulating hormone is used to induce superovulation in *in vitro* fertilization (IVF) which is an infertility treatment. In addition, it has been reported that in a case of solid cancer, FSH receptor is highly expressed in tumor vascular endothelium and is involved in neovascular regeneration; and therefore, once antagonists of FSH and FSH receptor are developed, such antagonists may also be used for anticancer therapy (N Engl J Med., 363 (17): 1621-1630, 2010).

Angiopoietin, a vascular growth factor, is directly involved in angiogenesis and regulates microvascular permeability, vasodilation, and vasoconstriction by signaling muscle cells surrounding vessels (BMC Infect Dis., 10: 143, 2010; Cancer Lett., 328 (1): 18-26, 2013). Four types of angiopoietins, ANGPT1, ANGPT2, ANGPT3, and ANGPT4, are known (Proc Natl Acad Sci USA., 96 (5): 1904-1909, 1999). Among these, angiopoietin-1 is a protein that plays an important role in vascular development and angiogenesis and is encoded by ANGPT1 gene. All angiopoietins bind to an endothelial cell-specific tyrosine-protein kinase receptor, and play an important role in mediating interactions between the endothelium and surrounding matrix and mesenchyme. Angiopoietin also contributes to vascular maturation and stability and is involved in early development of the heart (Cell Res., 13 (5): 309-317, 2003).

### Technical Problem

An object of the present invention is to provide a method for prolonging the half-life of a protein.

Another object of the present invention is to provide a protein with an extended half-life, the protein being obtained by substitution of at least one lysine residue present in the amino acid sequence thereof.

In addition, yet another object of the present invention is to provide a pharmaceutical composition comprising the protein with an extended half-life.

### Solution to Problem

In order to achieve the above objects, the present invention provides a method for prolonging the half-life of a protein, comprising substituting at least one lysine residue present in the amino acid sequence of the protein.

In the present invention, a lysine residue of a protein may be substituted with a conservative amino acid. In the present invention, "conservative amino acid substitution" means that an amino acid residue is substituted with another amino acid residue having a side chain with similar chemical properties, for example, charge or hydrophobicity. In general, conservative amino acid substitution does not substantially alter functional properties of a protein. Examples of groups of amino acids having side chains with similar chemical properties include 1) aliphatic side chains: glycine, alanine, valine, leucine, and isoleucine; 2) aliphatic-hydroxyl side chains: serine and threonine; 3) amide-containing side chains: asparagine and glutamine; 4) aromatic side chains: phenylalanine, tyrosine, and tryptophan; 5) basic side chains: lysine, arginine, and histidine; 6) acidic side chains: aspartate and glutamate; and 7) sulfur-containing side chains: cysteine and methionine.

In the present invention, a lysine residue of a protein may be substituted with an arginine or histidine residue containing a basic side chain, and is preferably substituted with an arginine residue.

### Advantageous Effects of Invention

According to the present invention, a protein obtained by substitution of at least one lysine residue, which is present in the amino acid sequence thereof, with arginine may have an extended half-life, and thus remain in the body for a long time.

### Brief Description of Drawings

FIG. 1 illustrates a structure of EGF expression vector.
FIG. 2 illustrates a size of EGF gene and PCR results thereof.
FIG. 3 illustrates protein expression through EGF plasmid in HEK-293T cells.
FIG. 4 illustrates degradation pathway of EGF through ubiquitination assay.
FIG. 5 illustrates ubiquitination levels of substituted EGFs, in which a lysine residue had been substituted with an arginine residue, as compared with the wild type.
FIG. 6 illustrates changes in half-life of EGF after treatment with the protein synthesis inhibitor cycloheximide (CHX).
FIG. 7 illustrates results for effects such as JAK-STAT, PI3K-AKT, and MAPK/ERK signal transduction.
FIG. 8 illustrates structures of PDGFA and PDGFB expression vectors.
FIG. 9 illustrates sizes of PDGFA and PDGFB genes and PCR results thereof.
FIG. 10 illustrates protein expression through PDGFA and PDGFB plasmids in HEK-293T cells.
FIG. 11 illustrates degradation pathways of PDGFA and PDGFB through ubiquitination assay.
FIG. 12 illustrates ubiquitination levels of substituted PDGFA and PDGFB, in each of which a lysine residue has been substituted with an arginine residue, as compared with each wild type.
FIGS. 13, 14, and 15 illustrate changes in half-life of PDGFA and PDGFB after treatment with the protein synthesis inhibitor cycloheximide (CHX).
FIG. 16 illustrates results for effects such as JAK-STAT, PI3K-AKT, and MAPK/ERK signal transduction.
FIG. 17 illustrates a structure of GM-CSF expression vector.
FIG. 18 illustrates a size of GM-CSF gene and PCR results thereof.
FIG. 19 illustrates protein expression through GM-CSF plasmid in HEK-293T cells.
FIG. 20 illustrates degradation pathway of GM-CSF through ubiquitination assay.
FIG. 21 illustrates ubiquitination levels of substitutued GM-CSF, in which a lysine residue had been substituted with an arginine residue, as compared with the wild type.
FIGS. 22 and 23 illustrate changes in half-life of GM-CSF after treatment with the protein synthesis inhibitor cycloheximide (CHX).
FIG. 24 illustrates results for effects such as JAK-STAT, PI3K-AKT, and MAPK/ERK signal transduction.
FIG. 25 illustrates structures of FSH-α and FSH-β expression vectors.
FIG. 26 illustrates sizes of FSH-α and FSH-β genes and PCR results thereof.
FIG. 27 illustrates protein expression through FSH-α and FSH-β plasmids in HEK-293T cells.
FIG. 28 illustrates degradation pathways of FSH-α and FSH-β through ubiquitination assay.
FIG. 29 illustrates ubiquitination levels of substitutued FSH-α and FSH-β, in each of which a lysine residue has been substituted with an arginine residue, as compared with each wild type.
FIGS. 30, 31, and 32 illustrate changes in half-life of FSH-α and FSH-β after treatment with the protein synthesis inhibitor cycloheximide (CHX).
FIG. 33 illustrates results for effects such as JAK-STAT, PI3K-AKT, and MAPK/ERK signal transduction.
FIG. 34 illustrates a structure of ANGPT-1 expression vector.
FIG. 35 illustrates a size of ANGPT-1 gene and PCR results thereof.
FIG. 36 illustrates protein expression through ANGPT-1 plasmid in HEK-293T cells.
FIG. 37 illustrates degradation pathway of ANGPT-1 through ubiquitination assay.
FIG. 38 illustrates ubiquitination levels of substitutued ANGPT-1, in which a lysine residue had been substituted with an arginine residue, as compared with the wild type.
FIGS. 39 and 40 illustrate changes in half-life of ANGPT-1 after treatment with the protein synthesis inhibitor cycloheximide (CHX).
FIG. 41 illustrates results for effects such as PI3K-AKT and MAPK/ERK signal transduction.

### Detailed Description of Invention

In an embodiment of the present invention, the protein is EGF. In the amino acid sequence of EGF represented by SEQ ID NO: 1, at least one of the 28^{th} and 48^{th} lysine residues from the N-terminus is substituted with an arginine residue. As a result, there are provided the EGF with an extended half-life, and a pharmaceutical and/or cosmetic composition for cell growth, skin and hair regeneration, and/or treatment, comprising the same (Exp Cell Res., 284 (1): 2-13, 2003; J Dermatol Sci., 72 (2): 81-86, 2013).

In another embodiment of the present invention, the protein is PDGFA. In the amino acid sequence of PDGFA represented by SEQ ID NO: 6, at least one of the 160^{th}, 165^{th}, and 206^{th} lysine residues from the N-terminus is substituted with an arginine residue. In addition, in yet another embodiment of the present invention, the protein is PDGFB. In the amino acid sequence of PDGFB represented by SEQ ID NO: 7, at least one of the 162^{nd}, 167^{th}, and 179^{th} lysine residues from the N-terminus is substituted with an arginine residue. Accordingly, there are provided the platelet-derived growth factor with an extended half-life, and a pharmaceutical and/or cosmetic composition for cell growth, angiogenesis, and recovery from chronic ulcers and bone loss, comprising the same.

In yet another embodiment of the present invention, the protein is GM-CSF. In the amino acid sequence of GM-CSF represented by SEQ ID NO: 20, at least one of the 89^{th}, 91^{st}, and 102^{nd} lysine residues from the N-terminus is substituted with an arginine residue. Accordingly, there are provided the GM-CSF with an extended half-life, and a pharmaceutical composition for prevention of neutropenia and/or prevention and/or treatment of an immune disease and/or cancer including solid cancer and hematological cancer and/or rheumatoid arthritis, comprising the same.

In still yet another embodiment of the present invention, the protein is FSH. In the amino acid sequence of FSH-α represented by SEQ ID NO: 27, at least one of the 75^{th}, 99^{th}, and 115^{th} lysine residues from the N-terminus is substituted with an arginine residue. In addition, in still yet another embodiment of the present invention, the protein is FSH-β. In the amino acid sequence of FSH-β represented by SEQ ID NO: 28, at least one of the 67^{th}, 104^{th}, and 128^{th} lysine residues from the N-terminus is substituted with an arginine residue. Accordingly, there are provided the FSH with an extended half-life, and an infertility therapeutic agent for inducing superovulation and/or pharmaceutical composition for treatment of solid cancer, comprising the same.

In still yet another embodiment of the present invention, the protein is ANGPT-1. In the amino acid sequence of ANGPT-1 represented by SEQ ID NO: 41, at least one of the 175^{th}, 216^{th}, and 414^{th} lysine residues from the N-terminus is substituted with an arginine residue. Accordingly, there is provided the ANGPT-1 with an extended half-life, and a pharmaceutical composition for treatment of diabetes, a cardiac disease, and/or sepsis, comprising the same.

In the present invention, site-directed mutagenesis was used to substitute a lysine residue, which is present in the amino acid sequence of a protein, with an arginine (R) residue. In this method, primers are prepared using a DNA sequence for which a specific mutation is to be induced, and then PCR is performed under specific conditions to construct plasmid DNA in which substitution of a specific amino acid residue had occurred.

In the present invention, a target protein was transfected into a cell line and precipitation was performed to identify the level of ubiquitination by immunoprecipitation assay. As a result of treatment with MG132 (proteasome inhibitor) reagent, it was identified that due to an increase in level of ubiquitination, the target protein undergoes degradation pathway caused by the ubiquitin-proteasome system.

In the present invention, the pharmaceutical composition may be delivered into the body in various routes including oral, transcutaneous, subcutaneous, intravenous, or intramuscular administration, and may be administered in an injectable preparation. In addition, the pharmaceutical composition of the present invention may be formulated according to methods well known to those of skill in the art such that rapid release, delayed release, or slow release is ensured after the pharmaceutical composition is administered according to the above method. The formulation may include tablets, pills, powders, sachets, elixirs, suspensions, emulsions, solutions, syrups, aerosols, soft and hard gelatin capsules, sterile injectable solutions, sterile packaged powders, and the like. Suitable carriers, excipients, and diluents include lactose, dextrose, sucrose, mannitol, xylitol, erythritol, maltitol, carbohydrates (starches), gum acacia, alginates, gelatin, calcium phosphate, calcium silicate, cellulose, methyl cellulose, microcrystalline cellulose, polyvinyl pyrrolidone, water, methylhydroxybenzoates, propylhydroxybenzoates, talc, magnesium stearate, and mineral oil. In addition, the formulation may further comprise fillers, anti-agglutinating agents, lubricating agents, wetting agents, flavoring agents, emulsifiers, preservatives, and the like.

In the present invention, the meaning of "a,", "an," and "the" includes plural reference unless the context clearly dictates otherwise. In addition, in the present invention, the terms such as "composed of," "having," and "consisting of" are interpreted as having a similar meaning to "comprising." In the present invention, "physiologically active (poly)peptide or protein" means a (poly)peptide or protein that exhibits useful biological activity when administered to a mammal including a human.

Hereinafter, the present invention will be described in more detail by way of examples. The following examples are given only for illustrating the present invention, and the present invention is not limited by the following examples.

### Example 1: Ubiquitination assay of EGF protein, and identification of extended half-life thereof and intracellular signal transduction thereof

### 1. Cloning into expression vector and identification of protein expression

### (1) Cloning into expression vector

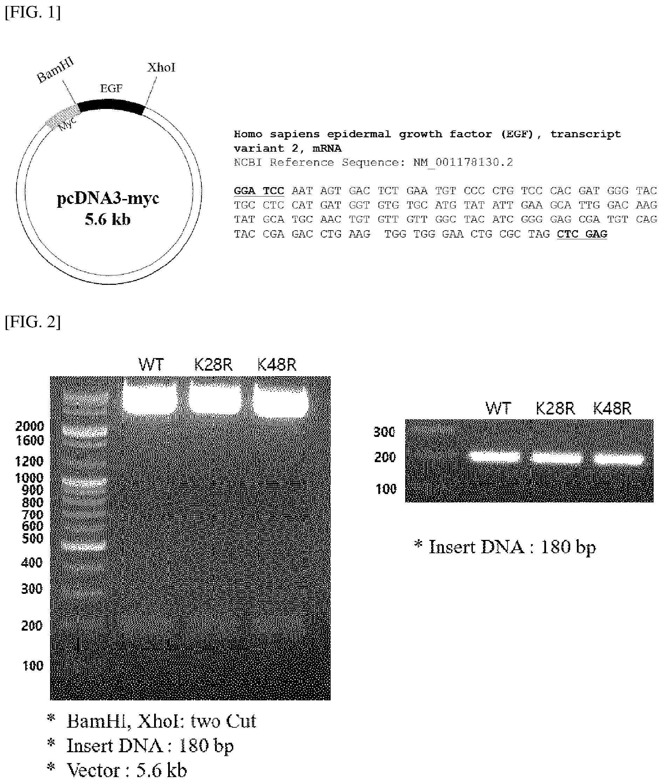

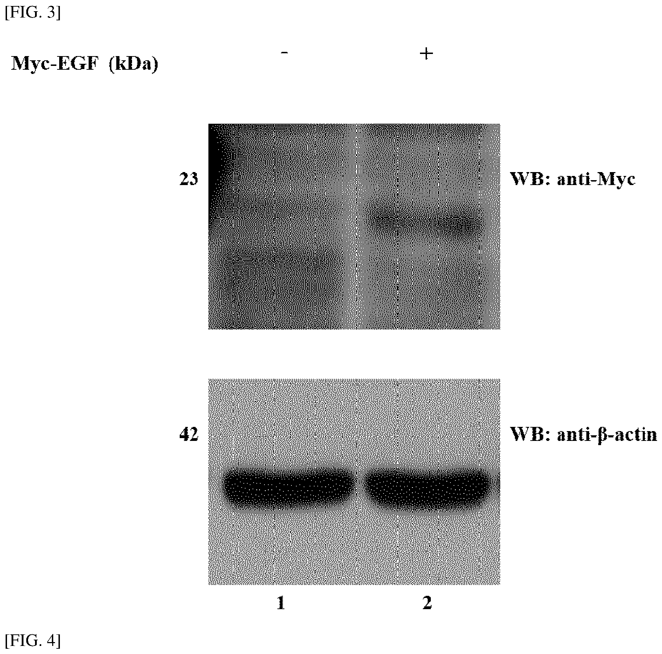

In order to clone EGF, purified RNA was extracted from HeLa cells (ATCC, CRM-CCL-2™) using Trizol and chloroform. Then, single-stranded cDNA was synthesized using the SuperScript™ First-Strand cDNA Synthesis System (Invitrogen, Grand Island, NY). Using the synthesized cDNA as a template, EGF was amplified by polymerase chain reaction. EGF DNA amplification products and pcDNA3-myc (5.6kb) were fragmented with restriction enzymes BamHI and Xhol, and then conjugation and cloning were performed (FIG. 1, amino acid sequence of EGF: SEQ ID NO: 1). The results were identified by cleavage with restriction enzymes and then agarose gel electrolysis (FIG. 2). In addition, the underlined and bold letters on the nucleotide sequence of FIG. 1 were primer sets used when polymerase chain reaction is performed to identify the cloned sites again, and the results were also identified by agarose gel electrophoresis (FIG. 2). The polymerase chain reaction condition was as follows: After initial denaturation at 94°C for 3 minutes, 25 cycles of denaturation at 94°C for 30 seconds, annealing at 52°C for 30 seconds, and extension at 72°C for 30 seconds were used, followed by reaction at 72°C for 10 minutes. In order to identify whether the DNA thus produced is properly expressed as a protein, expression of myc present in the pcDNA3-myc vector shown in the map of FIG. 1 was checked through Western blotting using an anti-myc (9E10, Santa Cruz Biotechnology, sc-40) antibody. Through this, it was identified that the EGF protein bound to myc is well expressed, and a blot identified as actin showed that an adequate amount of EGF has been loaded on myc (FIG. 3).

### (2) Substitution of lysine (K) residue

Site-directed mutagenesis was used to substitute a lysine residue with arginine (R), and primers (EGF K28R FP 5'-GAAGCATTGGACAGGTATGCATGCAAC-3' (SEQ ID NO: 2), RP 5'-GTTGCATGCATACCTGTCCAATGCTTC-3' (SEQ ID NO: 3), EGF K48R FP 5'-TACCGAGACCTGAGGTGGTGGGAACTG-3' (SEQ ID NO: 4), RP 5'-CAGTTCCCACCACCTCAGGTCTCGGTA-3' (SEQ ID NO: 5)) were prepared using a DNA sequence for which a specific mutation is to be induced; and then PCR was performed to construct plasmid DNAs in which substitution of a specific amino acid residue had occurred. Using pcDNA3-myc-EGF as a template, plasmid DNAs, in which a lysine residue had been substituted with arginine (K → R), were constructed (Table 1).

**[Table 1]**

| Lysine (K) residue site | EGF construct in which lysine (K) has been substituted with arginine (R) |
|---|---|
| 28 | pcDNA3-myc-EGF (K28R) |
| 48 | pcDNA3-myc-EGF (K48R) |
| 28 + 48 | pcDNA3-myc-EGF (K28R + K48R) |

### 2. In vivo ubiquitination assay

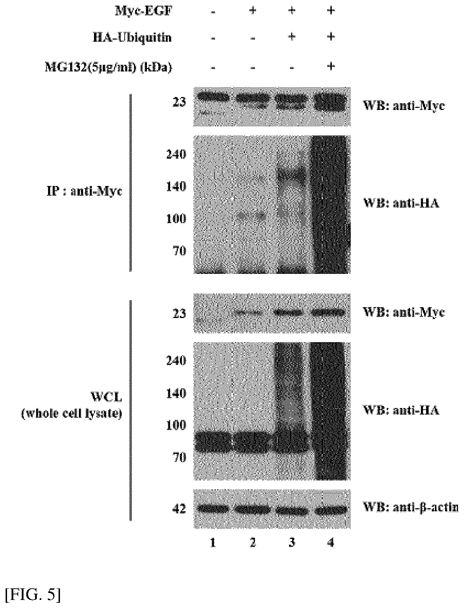

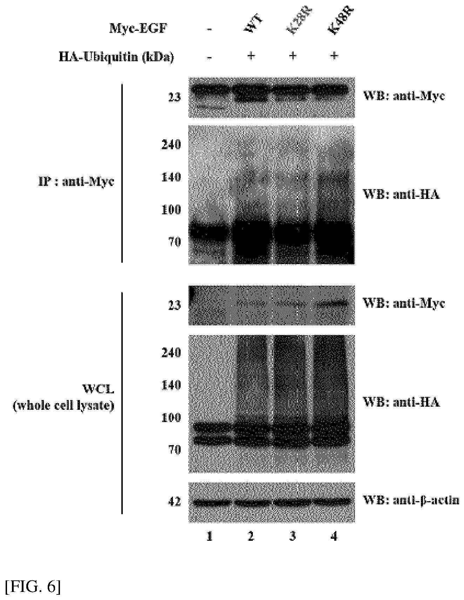

Plasmids encoding pcDNA3-myc-EGF WT and pMT123-HA-ubiquitin (J Biol Chem., 279 (4), 2368-2376, 2004; Cell Research, 22, 873-885, 2012; Oncogene, 22, 1273-1280, 2003; Cell, 78, 787-798,1994) were used to transfect HEK 293T cells (ATCC, CRL-3216). In order to identify a ubiquitination process, 3 µg of pcDNA3-myc-EGF WT and 1 µg of pMT123-HA-ubiquitin DNA were co-transfected into the cells; and, after 24 hours, the cells were subjected to treatment with MG132 (proteasome inhibitor, 5 µg/ml, Sigma Aldrich) for 6 hours, followed by immunoprecipitation assay (FIG. 4). In addition, in order to compare the level of ubiquitination between WT and the substituted ones, 3 µg of each of pcDNA3-myc-EGF WT, substituted pcDNA3-myc-EGF (K28R), and substituted pcDNA3-myc-EGF (K48R), and 1 µg of pMT123-HA-ubiquitin DNA were co-transfected into HEK 293T cells (ATCC, CRL-3216); and, after 24 hours, immunoprecipitation assay was performed (FIG. 5).

Protein samples obtained for immunoprecipitation were dissolved in lysis buffer (1% Triton X, 150 mM NaCl, 50 mM Tris-HCl, pH 8, and 1 mM phenylmethanesulfonyl fluoride (PMSF)), and then mixed with an anti-myc (9E10) primary antibody (Santa Cruz Biotechnology, sc-40). Incubation was performed overnight at 4°C. Immunoprecipitates were separated by reaction at 4°C for 2 hours using Protein A/G beads (Santa Cruz Biotechnology). Then, washing with lysis buffer was performed twice. The resultant was mixed with 2X SDS buffer, and then heated at 100°C for 7 minutes. Then, SDS-PAGE was performed to separate protein samples. The separated protein was transferred to a polyvinylidene difluoride (PVDF) membrane (Millipore), and then development was performed with an ECL system (Western blot detection kit, AbFrontier, Seoul, Korea) using anti-myc (9E10, Santa Cruz Biotechnology, sc-40), a blocking solution containing, at a weight ratio of 1:1,000, anti-HA (Santa Cruz Biotechnology, sc-7392) and anti-β-actin (Santa Cruz Biotechnology, sc-47778), and an anti-mouse (peroxidase-labeled antibody to mouse IgG (H+L), KPL, 074-1806) secondary antibody. As a result, in a case where immunoprecipitation is performed with anti-myc (9E10, sc-40), as ubiquitin binds to the pcDNA3-myc-EGF WT so that polyubiquitination is formed, a smear of ubiquitin was detected, and thus dark bands appeared (FIG. 4, lanes 3 and 4). In addition, in a case of being treated with MG132 (proteasome inhibitor, 5 µg/ml) for 6 hours, polyubiquitination formation increased, and thus a darker band appeared where ubiquitin is detected (FIG. 4, lane 4). These results suggest that EGF binds to ubiquitin and polyubiquitination occurs via the ubiquitin-proteasome system. In addition, the substituted pcDNA3-myc-EGF (K28R) exhibited a band lighter than WT. This indicates that ubiquitin failed to bind to this substituted one so that less ubiquitin was detected (FIG. 5, lane 3).

### 3. Identification of half-life of EGF by protein synthesis inhibitor cycloheximide (CHX)

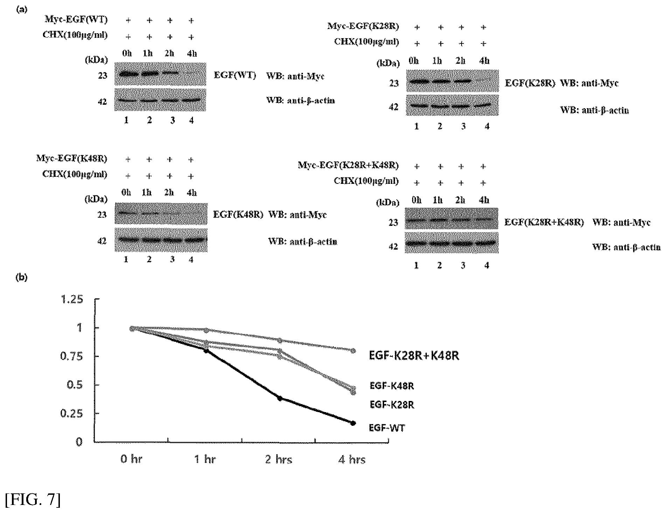

3 µg of each of pcDNA3-myc-EGF WT, substituted pcDNA3-myc-EGF (K28R), substituted pcDNA3-myc-EGF (K48R), and substituted pcDNA3-myc-EGF (K28R + K48R) was transfected into HEK 293T cells. 48 hours after transfection, treatment with the protein synthesis inhibitor cycloheximide (CHX) (Sigma-Aldrich) (100 µg/ml) was performed and the half-life of each of the EGFs was measured over 1 hour, 2 hours, and 4 hours. As a result, it was identified that degradation of human EGFs is suppressed (FIG. 6). The human EGF has a half-life of shorter than 2 hours. On the other hand, the human substitutued EGF (K28R), substitutued EGF (K48R), or substitutued EGF (K28R + K48R) has a half-life of equal to or longer than 4 hours, which is longer than WT. These results are graphically shown (FIG. 6).

### 4. Identification of intracellular signal transduction by EGF and substituted EGFs

It has been reported that EGF is a growth factor and activates Erk1/2 through Ras-Raf-MEK1/2-Erk signaling (Journal of Cell Science 117, 4619-4628, 2004).

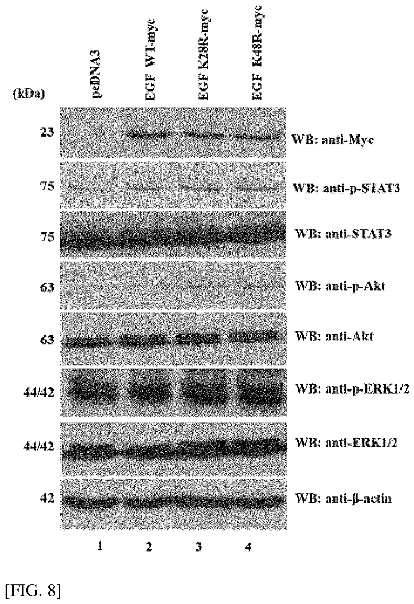

In the present example, intracellular signal transduction processes by EGF and substituted EGFs were identified. First, 3 µg of each of pcDNA3-myc-EGF WT, substituted pcDNA3-myc-EGF (K28R), and substituted pcDNA3-myc-EGF (K48R) was used to transfect HeLa cells. 2 days after transfection, proteins were extracted from the cells and respectively quantified. Western blotting was performed to identify intracellular signal transduction processes. To this end, each of the proteins, isolated from the HeLa cells transfected with each of the pcDNA3-myc-EGF WT, the substituted pcDNA3-myc-EGF (K28R), and the substituted pcDNA3-myc-EGF (K48R), was transferred to a polyvinylidene difluoride (PVDF) membrane, and then development was performed with an ECL system (Western blot detection kit, AbFrontier, Seoul, Korea) using anti-myc (9E10, Santa Cruz Biotechnology, sc-40), anti-STAT3 (Santa Cruz Biotechnology, sc-21876), anti-phospho-STAT3 (Y705, Cell Signaling Technology 9131S), anti-AKT (H-136, Santa Cruz Biotechnology, sc-8312), anti-phospho-AKT (S473, Cell Signaling Technology 9271S), anti-Erk1/2 (9B3, AbFrontier LF-MA0134), a blocking solution containing, at a weight ratio of 1:1,000 to 1:3,000, anti-phospho-Erk1/2 (Thr202/Tyr204, AbFrontier LF-PA0090) and anti-β-actin (Santa Cruz Biotechnology, sc-47778), and anti-rabbit (goat anti-rabbit IgG-HRP, Santa Cruz Biotechnology, sc-2004) and anti-mouse (peroxidase-labeled antibody to mouse IgG (H+L), KPL, 074-1806) secondary antibodies. As a result, the substituted pcDNA3-myc-EGF (K28R) and the substituted pcDNA3-myc-EGF (K48R) exhibited, within HeLa cells, phospho-STAT3, phospho-AKT, and phospho-Erk1/2 signal transduction whose levels are the same or increased as compared with the pcDNA3-myc-EGF WT (FIG. 7).

### Example 2: Ubiquitination assay of PDGF protein, and identification of extended half-life thereof and intracellular signal transduction thereof

### 1. Cloning into expression vector and identification of protein expression

### (1) Cloning into expression vector

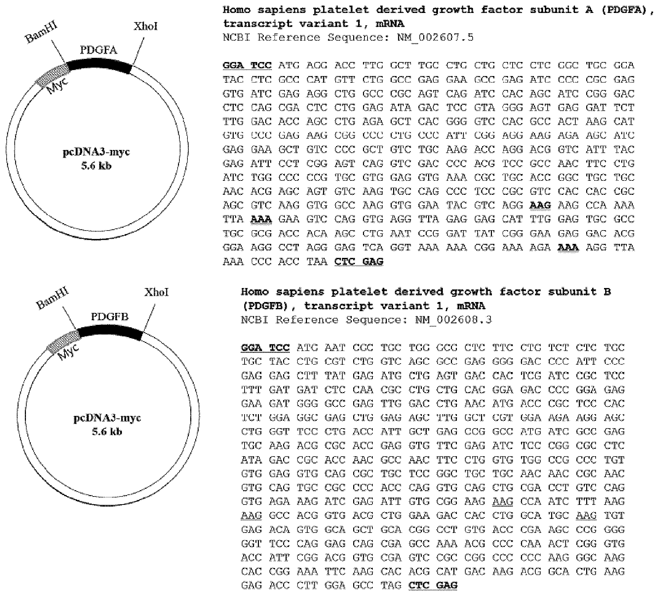

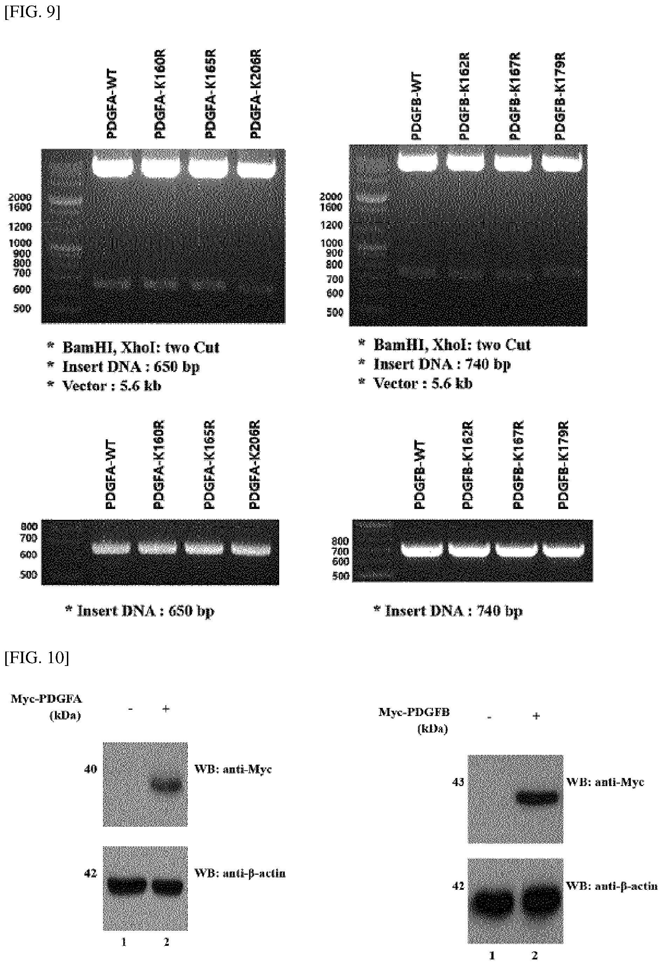

PDGFA and PDGFB DNA amplification products obtained by polymerase chain reaction, and pcDNA3-myc (5.6 kb) were fragmented with restriction enzymes BamHI and XhoI. Then, conjugation and cloning were performed (FIG. 8, amino acid sequences of PDGFA and PDGFB: SEQ ID NO: 6 and SEQ ID NO: 7). The results were identified by cleavage with restriction enzymes and then agarose gel electrolysis (FIG. 9). In addition, the underlined and bold letters on the nucleotide sequence of FIG. 8 were portions of primer sets used when polymerase chain reaction is performed to identify the cloned sites again, and the results were also identified by agarose gel electrophoresis (FIG. 9). The polymerase chain reaction condition was as follows: After initial denaturation at 94°C for 3 minutes, 25 cycles of denaturation at 94°C for 30 seconds, annealing at 60°C for 30 seconds, and extension at 72°C for 45 seconds were used, followed by reaction at 72°C for 10 minutes. In order to identify whether the DNA thus produced is properly expressed as a protein, expression of myc present in the pcDNA3-myc vector shown in the map of FIG. 8 was checked through Western blotting using an anti-myc (9E10, Santa Cruz Biotechnology, sc-40) antibody. It was identified that the PDGFA and PDGFB proteins bound to myc are well expressed, and a blot identified as actin showed that adequate amounts of PDGFA and PDGFB have been loaded on myc (FIG. 10).

### (2) Substitution of lysine (K) residue

Site-directed mutagenesis was used to substitute a lysine residue with arginine (R), and primers (PDGFA K160R FP 5'-GAATACGTCAGGAGGAAGCCAAAATTA-3' (SEQ ID NO: 8), RP 5'-TAATTTTGGCTTCCTCCTGACGTATTC-3' (SEQ ID NO: 9); PDGFA K165R FP 5'-AAGCCAAAATTAAGAGAAGTCCAGGTG-3' (SEQ ID NO: 10), RP 5'-CACCTGGACTTCTCTTAATTTTGGCTT-3' (SEQ ID NO: 11); PDGFA K206R FP 5'-AAACGGAAAAGAAGAAGGTTAAAACCC-3' (SEQ ID NO: 12), RP 5'-GGGTTTTAACCTTCTTCTTTTCCGTTT-3' (SEQ ID NO: 13), (PDGFB K162R FP 5'-ATTGTGCGGAAGAGGCCAATCTTT-3' (SEQ ID NO: 14), RP 5'-AAAGATTGGCCTCTTCCGCACAAT-3' (SEQ ID NO: 15); PDGFB K167R FP 5'-CCAATCTTTAAGAGGGCCACGGTG-3' (SEQ ID NO: 16), RP 5'-CACCGTGGCCCTCTTAAAGATTGG-3' (SEQ ID NO: 17); PDGFB K179R FP 5'-CACCTGGCATGCAGGTGTGAGACA-3' (SEQ ID NO: 18), RP 5'-TGTCTCACACCTGCATGCCAGGTG-3' (SEQ ID NO: 19)) were prepared using a DNA sequence for which a specific mutation is to be induced; and then PCR was performed to construct plasmid DNAs in which substitution of a specific amino acid residue had occurred. Using pcDNA3-myc-PDGFA as a template, three plasmid DNAs, in which a lysine residue had been substituted with arginine (K → R), were constructed (Table 2).

**[Table 2]**

| Lysine (K) residue site | PDGFA and PDGFB constructs, in each of which lysine (K) has been substituted with arginine (R) |
|---|---|
| 160 | pcDNA3-myc-PDGFA (K160R) |
| 165 | pcDNA3-myc-PDGFA (K165R) |
| 206 | pcDNA3-myc-PDGFA (K206R) |
| 162 | pcDNA3-myc-PDGFB (K162R) |
| 167 | pcDNA3-myc-PDGFB (K167R) |
| 179 | pcDNA3-myc-PDGFB (K179R) |

### 2. In vivo ubiquitination assay

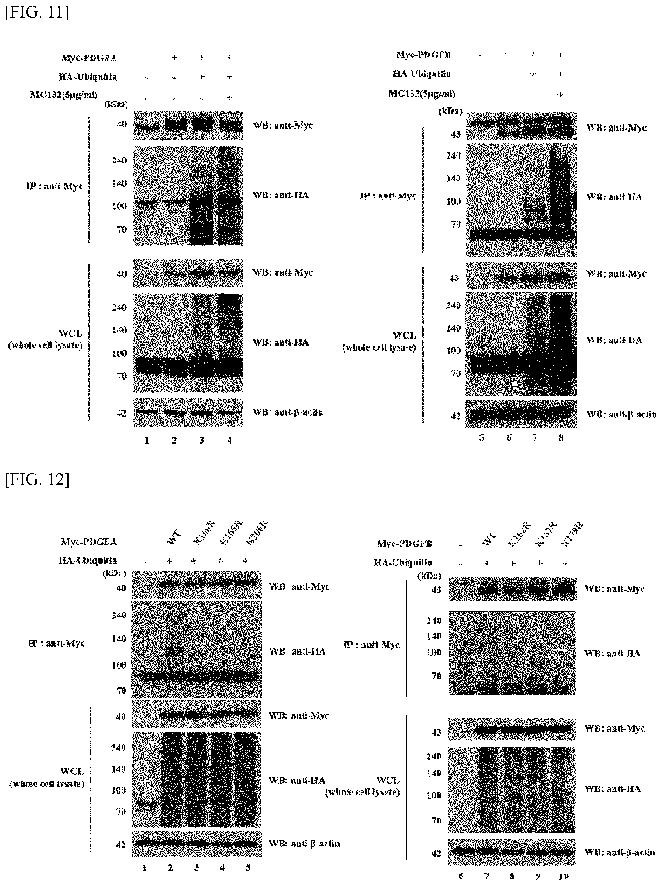

Plasmids encoding pcDNA3-myc-PDGFA WT and pMT123-HA-ubiquitin (J Biol Chem., 279 (4), 2368-2376, 2004; Cell Research, 22, 873-885, 2012; Oncogene, 22, 1273-1280, 2003; Cell, 78, 787-798, 1994) were used to transfect HEK 293T cells (ATCC, CRL-3216). In order to identify a ubiquitination process, 3 µg of pcDNA3-myc-PDGFA WT and 1 µg of pMT123-HA-ubiquitin DNA were co-transfected into the cells; and, after 24 hours, the cells were subjected to treatment with MG132 (proteasome inhibitor, 5 µg/ml) for 6 hours, followed by immunoprecipitation assay (FIG. 4). In addition, in order to compare the level of ubiquitination between WT and the substituted ones, 3 µg of each of pcDNA3-myc-PDGFA WT, substituted pcDNA3-myc-PDGFA (K160R), substituted pcDNA3-myc-PDGFA (K165R), and substituted pcDNA3-myc-PDGFA (K206R), or 3 µg of each of pcDNA3-myc-PDGFB WT, substituted pcDNA3-myc-PDGFB (K162R), substituted pcDNA3-myc-PDGFB (K167R), and substituted pcDNA3-myc-PDGFB (K179R), and 1 µg of pMT123-HA-ubiquitin DNA were co-transfected into HEK 293T cells (ATCC, CRL-3216); and, after 24 hours, immunoprecipitation assay was performed (FIG. 11).

Protein samples obtained for immunoprecipitation were dissolved in lysis buffer (1% Triton X, 150 mM NaCl, 50 mM Tris-HCl, pH 8, and 1 mM phenylmethanesulfonyl fluoride (PMSF)), and then mixed with an anti-myc (9E10) primary antibody (Santa Cruz Biotechnology, sc-40). Incubation was performed overnight at 4°C. Immunoprecipitates were separated by reaction at 4°C for 2 hours using Protein A/G beads (Santa Cruz Biotechnology). Then, washing with lysis buffer was performed twice. The resultant was mixed with 2X SDS buffer, and then heated at 100°C for 7 minutes. Then, SDS-PAGE was performed to separate protein samples. The separated protein was transferred to a polyvinylidene difluoride (PVDF) membrane (Millipore), and then development was performed with an ECL system (Western blot detection kit, AbFrontier, Seoul, Korea) using anti-myc (9E10, Santa Cruz Biotechnology, sc-40), a blocking solution containing, at a weight ratio of 1:1,000, anti-HA (Santa Cruz Biotechnology, sc-7392) and anti-β-actin (Santa Cruz Biotechnology, sc-47778), and an anti-mouse (peroxidase-labeled antibody to mouse IgG (H+L), KPL, 074-1806) secondary antibody. As a result, in a case where immunoprecipitation is performed with anti-myc (9E10, sc-40), as ubiquitin binds to the pcDNA3-myc-PDGFA WT and the pcDNA3-myc-PDGFB WT so that polyubiquitination is formed, a smear of ubiquitin was detected, and thus dark bands appeared (FIG. 4, lanes 3, 4, 7, and 8). In addition, in a case of being treated with MG132 (proteasome inhibitor, 5 µg/ml) for 6 hours, polyubiquitination formation increased, and thus darker bands appeared where ubiquitin is detected (FIG. 4, lanes 4 and 8). These results suggest that PDGFA and PDGFB bind to ubiquitin and polyubiquitination occurs via the ubiquitin-proteasome system. Also, the substituted pcDNA3-myc-PDGFA (K160R), the substituted pcDNA3-myc-PDGFA (K165R), the substituted pcDNA3-myc-PDGFA (K206R), the substituted pcDNA3-myc-PDGFB (K162R), the substituted pcDNA3-myc-PDGFB (K167R), or the substituted pcDNA3-myc-PDGFB (K179R) exhibited a band lighter than WT. This indicates that ubiquitin failed to bind to these substituted ones so that less ubiquitin was detected (FIG. 12, lanes 3 to 5 and 8 to 10).

### 3. Identification of half-life of PDGF by protein synthesis inhibitor cycloheximide (CHX)

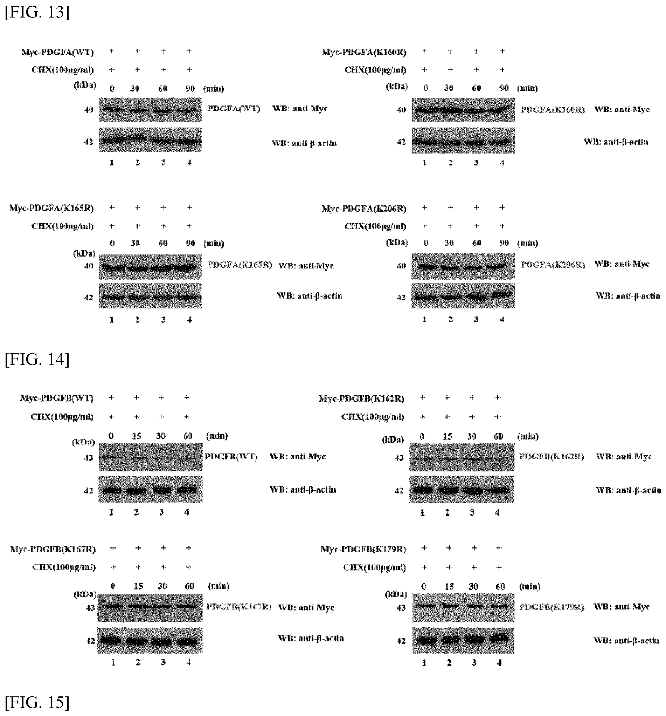

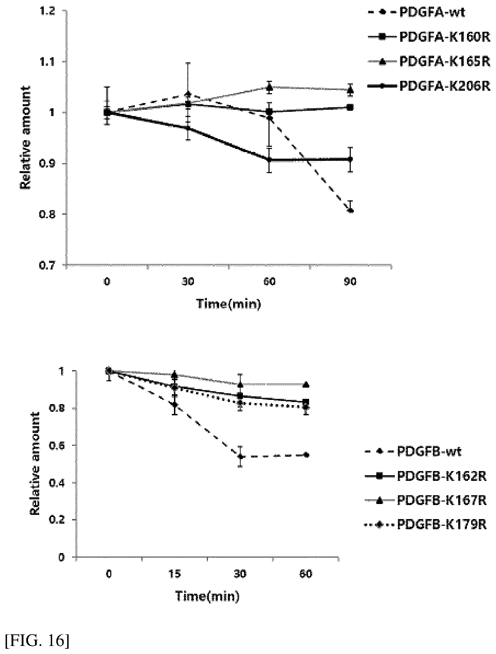

3 µg of each of pcDNA3-myc-PDGFA WT, substituted pcDNA3-myc-PDGFA (K160R), substituted pcDNA3-myc-PDGFA (K165R), and substituted pcDNA3-myc-PDGFA (K206R), or 3 µg of each of pcDNA3-myc-PDGFB WT, substituted pcDNA3-myc-PDGFB (K162R), substituted pcDNA3-myc-PDGFB (K167R), and substituted pcDNA3-myc-PDGFB (K179R) was transfected into HEK 293T cells. 48 hours after transfection, treatment with the protein synthesis inhibitor cycloheximide (CHX) (Sigma-Aldrich) (100 µg/ml) was performed; and the half-life of each of the PDGFAs was measured over 30, 60, and 90 minutes, and the half-life of each of the PDGFBs was measured over 15, 30 and 60 minutes. As a result, it was identified that degradation of human PDGFAs and PDGFBs is suppressed (FIG. 6). From the results, it can be seen that the human PDGFA degrades after 60 minutes, whereas the human substituted PDGFA (K160R), substituted PDGFA (K165R), and substituted PDGFA (K206R) do not degrade even after 90 minutes; and it can be seen that the human PDGFB degrades within 30 minutes, whereas the human substituted PDGFB (K162R), substituted PDGFB (K167R), and substituted PDGFB (K179R) do not degrade even after 60 minutes. Such results are graphically shown (FIGS. 13, 14, and 15).

### 4. Identification of intracellular signal transduction by PDGF and substituted PDGFs

It has been reported that PDGF is a growth factor and activates Erk1/2 through Ras-Raf-MEK1/2-Erk signaling (Journal of Cell Science 117, 4619-4628, 2004).

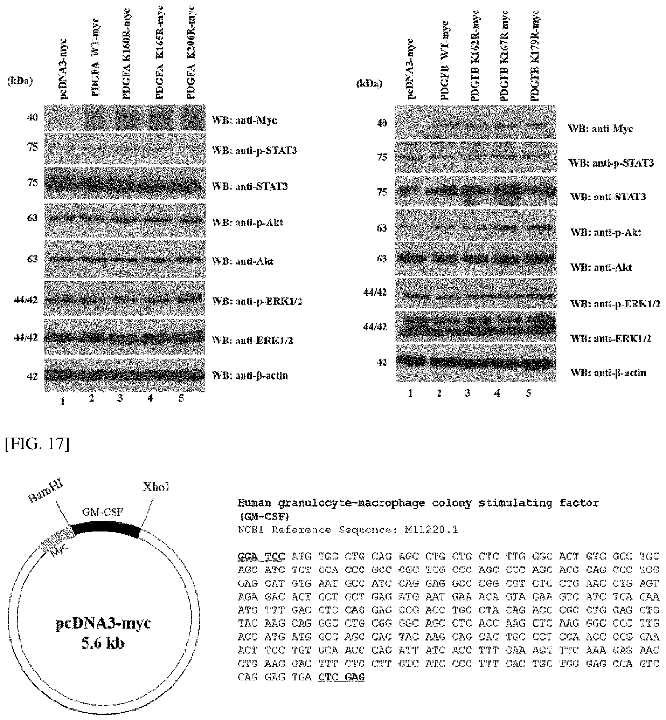

In the present example, intracellular signal transduction processes by PDGF and substituted PDGFs were identified. First, 3 µg of each of pcDNA3-myc-PDGFA WT, substituted pcDNA3-myc-PDGFA (K160R), substituted pcDNA3-myc-PDGFA (K165R), and substituted pcDNA3-myc-PDGFA (K206R), or 3 µg of each of pcDNA3-myc-PDGFB WT, substituted pcDNA3-myc-PDGFB (K162R), substituted pcDNA3-myc-PDGFB (K167R), and substituted pcDNA3-myc-PDGFB (K179R) was used to transfect HeLa cells. 2 days after transfection, proteins were extracted from the cells and respectively quantified. Western blotting was performed to identify intracellular signal transduction processes. To this end, each protein, isolated from the HeLa cells transfected with each of the pcDNA3-myc-PDGFA WT, the substituted pcDNA3-myc-PDGFA (K160R), the substituted pcDNA3-myc-PDGFA (K165R), and substituted pcDNA3-myc-PDGFA (K206R), or each of the pcDNA3-myc-PDGFB WT, the substituted pcDNA3-myc-PDGFB (K162R), the substituted pcDNA3-myc-PDGFB (K167R), and the substituted pcDNA3-myc-PDGFB (K179R), was transferred to a polyvinylidene difluoride (PVDF) membrane, and then development was performed with an ECL system (Western blot detection kit, AbFrontier, Seoul, Korea) using anti-myc (9E10, Santa Cruz Biotechnology, sc-40), anti-STAT3 (Santa Cruz Biotechnology, sc-21876), anti-phospho-STAT3 (Y705, Cell Signaling Technology 9131S), anti-AKT (H-136, Santa Cruz Biotechnology, sc-8312), anti-phospho-AKT (S473, Cell Signaling Technology 9271S), anti-Erk1/2 (9B3, AbFrontier LF-MA0134), a blocking solution containing, at a weight ratio of 1:1,000 to 1:3,000, anti-phospho-Erk1/2 (Thr202/Tyr204, AbFrontier LF-PA0090) and anti-β-actin (Santa Cruz Biotechnology, sc-47778), and anti-rabbit (goat anti-rabbit IgG-HRP, Santa Cruz Biotechnology, sc-2004) and anti-mouse (peroxidase-labeled antibody to mouse IgG (H+L), KPL, 074-1806) secondary antibodies. As a result, the substituted pcDNA3-myc-PDGFA (K160R), the substituted pcDNA3-myc-PDGFA (K165R), and the substituted pcDNA3-myc-PDGFA (K206R) exhibited, within HeLa cells, phospho-STAT signal transduction which is the same or increased as compared with the pcDNA3-myc-PDGFA WT; and the substituted pcDNA3-myc-PDGFB (K162R), the substituted pcDNA3-myc-PDGFB (K167R), and the substituted pcDNA3-myc-PDGFB (K179R) exhibited, within HeLa cells, phospho-AKT and phospho-Erk1/2 signal transduction (FIG. 16).

### Example 3: Ubiquitination assay of GM-CSF protein, and identification of extended half-life thereof and intracellular signal transduction thereof

### 1. Cloning into expression vector and identification of protein expression

### (1) Cloning into expression vector

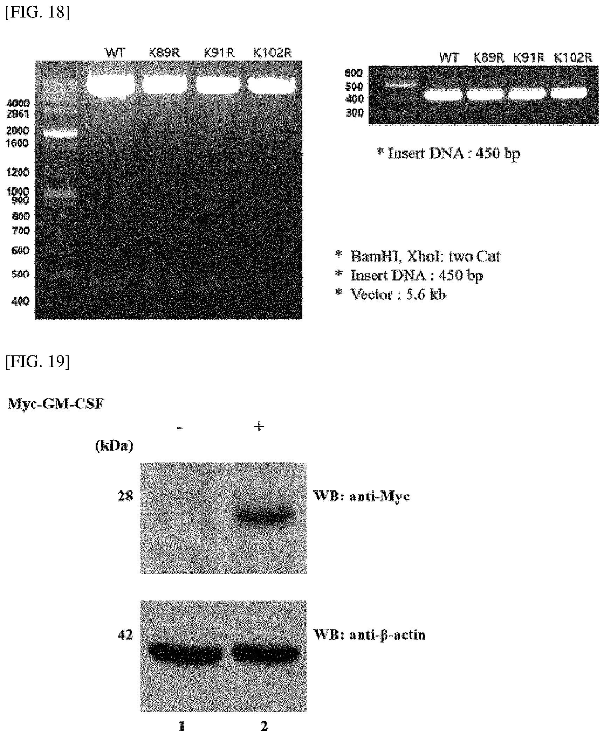

GM-CSF amplification products obtained by polymerase chain reaction, and pcDNA3-myc (5.6 kb) were fragmented with restriction enzymes BamHI and XhoI. Then, conjugation and cloning were performed (FIG. 17, amino acid sequence of GM-CSF: SEQ ID NO: 20). The results were identified by cleavage with restriction enzymes and then agarose gel electrolysis (FIG. 18). In addition, the underlined and bold letters on the nucleotide sequence of FIG. 17 were portions of primer sets used when polymerase chain reaction is performed to identify the cloned sites again, and the results were identified by agarose gel electrophoresis (FIG. 18). The polymerase chain reaction condition was as follows: After initial denaturation at 94°C for 3 minutes, 25 cycles of denaturation at 94°C for 30 seconds, annealing at 60°C for 30 seconds, and extension at 72°C for 30 seconds were used, followed by reaction at 72°C for 10 minutes. In order to identify whether the DNA thus produced is properly expressed as a protein, expression of myc present in the pcDNA3-myc vector shown in the map of FIG. 17 was checked through Western blotting using an anti-myc (9E10, sc-40) antibody. Through this, it was identified that the GM-CSF protein bound to myc is well expressed, and a blot identified as actin showed that an adequate amount of GM-CSF has been loaded on myc (FIG. 19).

### (2) Substitution of lysine (K) residue

Site-directed mutagenesis was used to substitute a lysine residue with arginine, and primers (GM-CSF K89R FP 5'-GGCAGCCTCACCAGGCTCAAGGGCC-3' (SEQ ID NO: 21), RP 5'-GGCCCTTGAGCCTGGTGAGGCTGCC-3' (SEQ ID NO: 22); GM-CSF K91R FP 5'-CTCACCAAGCTCAGGGGCCCCTTGACC-3' (SEQ ID NO: 23), RP 5'-GGTCAAGGGGCCCCTGAGCTTGGTGAG-3' (SEQ ID NO: 24); GM-CSF K102R FP 5'-GCTAGCCACTACAGACAGCACTGCCCT-3' (SEQ ID NO: 25), RP 5'-AGGGCAGTGCTGTCTGTAGTGGCTAGC-3' (SEQ ID NO: 26)) were prepared using a DNA sequence for which a specific mutation is to be induced; and then PCR was performed under a specific condition to construct plasmid DNAs in which substitution of a specific amino acid residue had occurred. Using pcDNA3-myc-GM-CSF as a template, three plasmid DNAs, in which a lysine residue had been substituted with arginine (K → R), were constructed (Table 3).

**[Table 3]**

| Lysine (K) residue site | GM-CSF construct in which lysine (K) has been substituted with arginine (R) |
|---|---|
| 89 | pcDNA3-myc-GM-CSF (K89R) |
| 91 | pcDNA3-myc-GM-CSF (K91R) |
| 102 | pcDNA3-myc-GM-CSF (K102R) |

### 2. In vivo ubiquitination assay

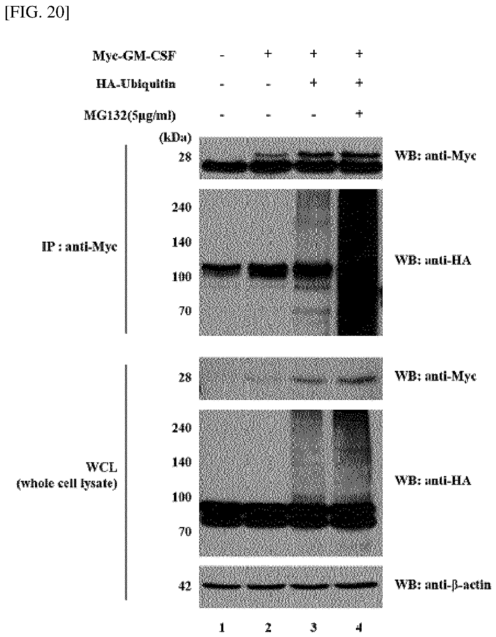

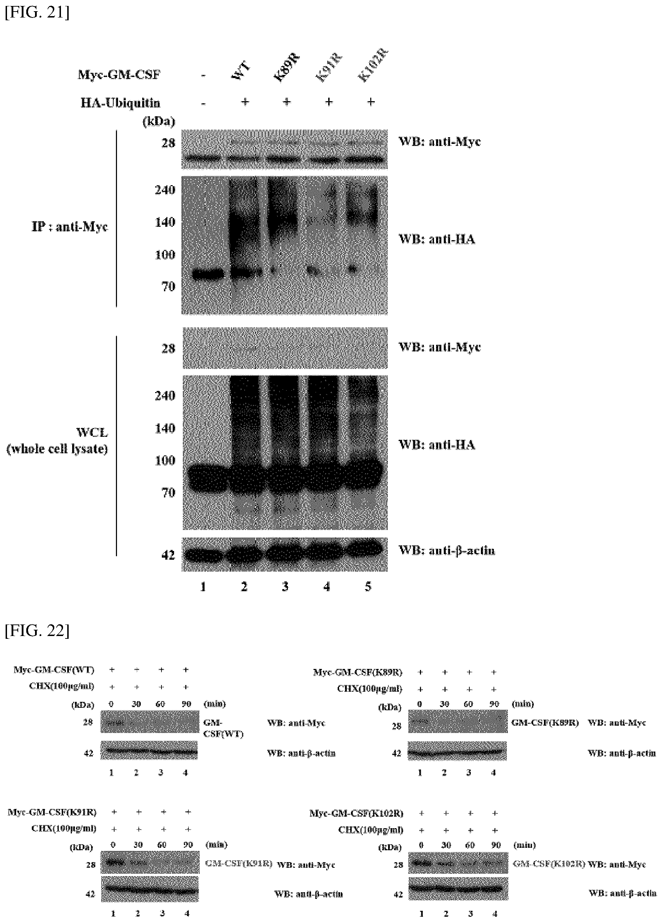

Plasmids encoding pcDNA3-myc-GM-CSF WT and pMT123-HA-ubiquitin DNA were used to transfect HEK 293T cells. In order to identify a ubiquitination process, 3 µg of pcDNA3-myc-GM-CSF WT and 1 µg of pMT123-HA-ubiquitin DNA were co-transfected into the cells; and, after 24 hours, the cells were subjected to treatment with MG132 (proteasome inhibitor, 5 µg/ml) for 6 hours, followed by immunoprecipitation assay (FIG. 20). In addition, in order to compare the level of ubiquitination between WT and the substituted ones, 3 µg of each of pcDNA3-myc-GM-CSF WT, substituted pcDNA3-myc-GM-CSF (K89R), substituted pcDNA3-myc-GM-CSF (K91R), and substituted pcDNA3-myc-GM-CSF (K102R), and 1 µg of pMT123-HA-ubiquitin DNA were co-transfected into HEK 293T cells (ATCC, CRL-3216); and, after 24 hours, immunoprecipitation assay was performed (FIG. 21).

Samples obtained for immunoprecipitation were dissolved in lysis buffer (1% Triton X, 150 mM NaCl, 50 mM Tris-HCl, pH 8, and 1 mM phenylmethanesulfonyl fluoride (PMSF)), and then mixed with an anti-myc (9E10) primary antibody. Incubation was performed overnight at 4°C. Immunoprecipitates were separated by reaction at 4°C for 2 hours using Protein A/G beads (Santa Cruz Biotechnology). Then, washing with lysis buffer was performed twice. In immunoblotting, the resultant was mixed with 2X SDS buffer, and then heated at 100°C for 7 minutes, followed by SDS-PAGE to separate protein samples. The separated protein was transferred to a PVDF membrane, and then development was performed with an ECL system (Western blot detection kit, AbFrontier, Seoul, Korea) using anti-myc (9E10, Santa Cruz Biotechnology, sc-40), a blocking solution containing, at a weight ratio of 1:1,000, anti-HA (Santa Cruz Biotechnology, sc-7392) and anti-β-actin (Santa Cruz Biotechnology, sc-47778), and an anti-mouse (peroxidase-labeled antibody to mouse IgG (H+L), KPL, 074-1806) secondary antibody.

As a result, in a case where immunoprecipitation is performed with anti-myc (9E10, sc-40), as ubiquitin binds to the pcDNA3-myc-GM-CSF WT so that polyubiquitination is formed, a smear of ubiquitin was detected, and thus dark bands appeared (FIG. 20, lanes 3 and 4). In addition, in a case of being treated with MG132 (proteasome inhibitor, 5 µg/ml) for 6 hours, polyubiquitination formation increased, and thus a darker band appeared where ubiquitin is detected (FIG. 20, lane 4). In addition, the substituted pcDNA3-myc-GM-CSF (K89R), the substituted pcDNA3-myc-GM-CSF (K91R), or the substituted pcDNA3-myc-GM-CSF (K102R) exhibited a band lighter than WT, and the pcDNA3-myc-GM-CSF WT, the substituted pcDNA3-myc-GM-CSF (K89R), the substituted pcDNA3-myc-GM-CSF (K91R), or the substituted pcDNA3-myc-GM-CSF (K102R) failed to bind with ubiquitin so that less ubiquitin was detected (FIG. 21, lanes 3, 4, and 5). The above results show that GM-CSF binds to ubiquitin and polyubiquitination occurs via the ubiquitin-proteasome system for its degradation.

### 3. Identification of half-life of GM-CSF by protein synthesis inhibitor cycloheximide (CHX)

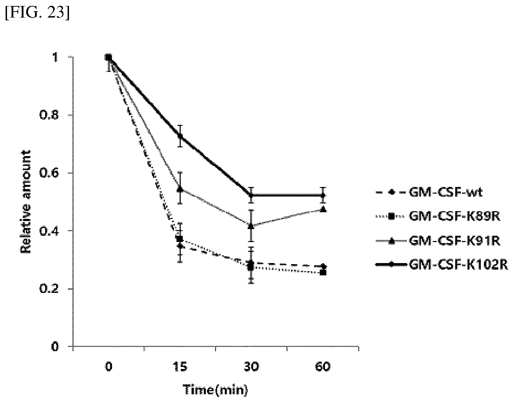

3 µg of each of pcDNA3-myc-GM-CSF WT, substituted pcDNA3-myc-GM-CSF (K89R), substituted pcDNA3-myc-GM-CSF (K91R), and substituted pcDNA3-myc-GM-CSF (K102R) was transfected into HEK 293T cells. 48 hours after transfection, treatment with the protein synthesis inhibitor cycloheximide (CHX) (Sigma-Aldrich) (100 µg/ml) was performed; and the half-life of each of the GM-CSFs was measured over 30, 60, and 90 minutes. As a result, it was identified that degradation of human GM-CSFs is suppressed (FIGS. 22 and 23). The human GM-CSF had a half-life of shorter than 30 minutes, whereas the human substituted pcDNA3-myc-GM-CSF (K91R) and the substituted pcDNA3-myc-GM-CSF (K102R) had half-lives of 60 minutes and equal to or longer than 90 minutes, respectively, which is longer than WT. These results are graphically shown (FIGS. 22 and 23).

### 4. Identification of intracellular signal transduction by GM-CSF and substituted GM-CSFs

It has been reported that GM-CSF has intracellular roles which are acted by JAK/STAT, MAPK, and PI3K/AKT signal transduction (Blood, 119 (15): 3383-3393, 2012).

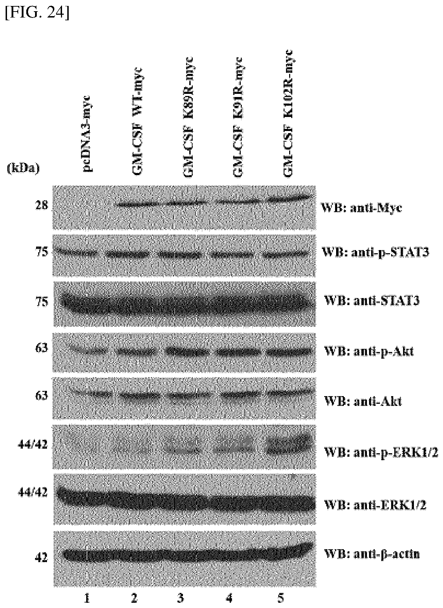

In the present example, intracellular signal transduction processes by GM-CSF and substituted GM-CSFs were identified. 3 µg of each of substituted pcDNA3-myc-GM-CSF (K89R), substituted pcDNA3-myc-GM-CSF (K91R), and substituted pcDNA3-myc-GM-CSF (K102R) was used to transfect HeLa cells. 2 days after transfection, proteins were extracted from the transfected cells and respectively quantified. Western blotting was performed to identify intracellular signal transduction processes. In this procedure, each protein, isolated from the HeLa cells transfected with each of the substituted pcDNA3-myc-GM-CSF (K89R), the substituted pcDNA3-myc-GM-CSF (K91R), and the substituted pcDNA3-myc-GM-CSF (K102R), was transferred to a polyvinylidene difluoride (PVDF) membrane, and then development was performed with an ECL system (Western blot detection kit, AbFrontier, Seoul, Korea) using anti-myc (9E10, Santa Cruz Biotechnology, sc-40), anti-STAT3 (Santa Cruz Biotechnology, sc-21876), anti-phospho-STAT3 (Y705, Cell Signaling Technology 9131S), anti-AKT (H-136, Santa Cruz Biotechnology, sc-8312), anti-phospho-AKT (S473, Cell Signaling Technology 9271S), anti-Erk1/2 (9B3, AbFrontier LF-MA0134), a blocking solution containing, at a weight ratio of 1:1,000 to 1:3,000, anti-phospho-Erk1/2 (Thr202/Tyr204, AbFrontier LF-PA0090) and anti-β-actin (Santa Cruz Biotechnology, sc-47778), and anti-rabbit (goat anti-rabbit IgG-HRP, Santa Cruz Biotechnology, sc-2004) and anti-mouse (peroxidase-labeled antibody to mouse IgG (H+L), KPL, 074-1806) secondary antibodies. As a result, the substituted pcDNA3-myc-GM-CSF (K89R), the substituted pcDNA3-myc-GM-CSF (K91R), and the substituted pcDNA3-myc-GM-CSF (K102R) exhibited, within HeLa cells, phospho-STAT and phospho-AKT signal transduction which is the same or increased as compared with the pcDNA3-myc-GM-CSF WT (FIG. 24).

### Example 4: Ubiquitination assay of FSH protein, and identification of extended half-life thereof and intracellular signal transduction thereof

### 1. Cloning into expression vector and identification of protein expression

### (1) Cloning into expression vector

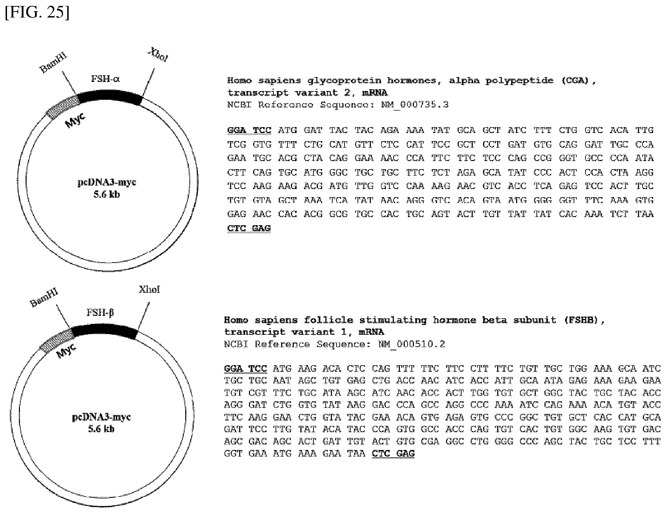

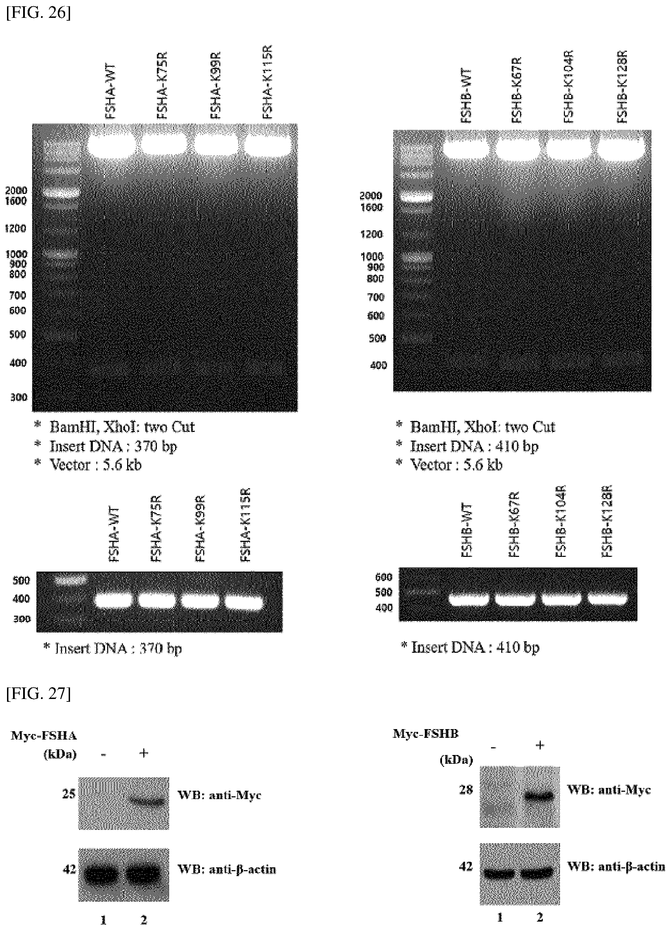

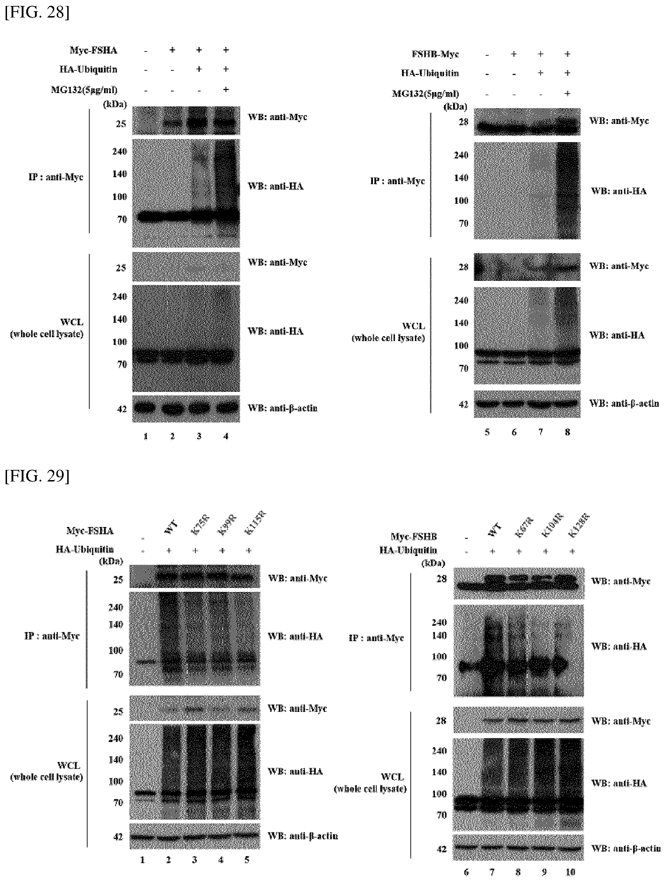

FSH-α and FSH-β DNA amplification products obtained by polymerase chain reaction, and pcDNA3-myc (5.6 kb) were fragmented with restriction enzymes BamHI and XhoI. Then, conjugation and cloning were performed (FIG. 25, amino acid sequence of FSH-α: SEQ ID NO: 27; and amino acid sequence of FSH-β: SEQ ID NO: 28). The results were identified by cleavage with restriction enzymes and then agarose gel electrolysis (FIG. 26). In addition, the underlined and bold letters on the nucleotide sequence of FIG. 25 were portions of primer sets used when polymerase chain reaction is performed to identify the cloned sites again, and the results were identified by agarose gel electrophoresis (FIG. 26). The polymerase chain reaction condition was as follows: After initial denaturation at 94°C for 3 minutes, 25 cycles of denaturation at 94°C for 30 seconds, annealing at 56°C for 30 seconds, and extension at 72°C for 1 minute were used, followed by reaction at 72°C for 10 minutes. In order to identify whether the DNA thus produced is properly expressed as a protein, expression of myc present in the pcDNA3-myc vector shown in the map of FIG. 29 was checked through Western blotting using an anti-myc (9E10, Santa Cruz Biotechnology, sc-40) antibody. It was identified that the FSH-α and FSH-β proteins bound to myc are well expressed, and a blot identified as actin showed that adequate amounts of FSH-α and FSH-β have been loaded on myc (FIG. 27).

### (2) Substitution of lysine (K) residue

Site-directed mutagenesis was used to substitute a lysine residue with arginine, and primers (FSH-α K75R FP 5'-ATGTTGGTCCAAAGGAACGTCACC-3' (SEQ ID NO: 29), RP 5'-GGTGACGTTCCTTTGGACCAACAT-3' (SEQ ID NO: 30), FSH-α K99R FP 5'-ATGGGGGGTTTCAGAGTGGAGAAC-3' (SEQ ID NO: 31), RP 5'-GTTCTCCACTCTGAAACCCCCCAT-3' (SEQ ID NO: 32), FSH-α K115R FP 5'-TGTTATTATCACAGATCTTAACTCGAG-3' (SEQ ID NO: 33), RP 5'-CTCGAGTTAAGATCTGTGATAATAACA-3' (SEQ ID NO: 34), FSH-β K67R FP 5'-GGCCCAAAATCCAGAGAACATGTACCTT-3' (SEQ ID NO: 35), RP 5'-AAGGTACATGTTCTCTGGATTTTGGGCC-3' (SEQ ID NO: 36), FSH-β K104R FP 5'-GTCACTGTGGCAGGTGTGACAGCGA-3' (SEQ ID NO: 37), RP 5'-TCGCTGTCACACCTGCCACAGTGAC-3' (SEQ ID NO: 38), FSH-β K128R FP 5'-GGTGAAATGAGAGAAACGCGTACGCGG-3' (SEQ ID NO: 39), RP 5'-CCGCGTACGCGTTTCTCTCATTTCACC-3' (SEQ ID NO: 40)) were prepared using a DNA sequence for which a specific mutation is to be induced; and then PCR was performed under a specific condition to construct plasmid DNAs in which substitution of a specific amino acid residue had occurred. Using pcDNA3-myc-FSH-α and pcDNA3-myc-FSH-β as templates, three plasmid DNAs, in which a lysine residue had been substituted with arginine (K → R), were constructed (Table 4).

**[Table 4]**

| Lysine (K) residue site | FSH-α, FSH-β constructs, in each of which lysine (K) has been substituted with arginine (R) |
|---|---|
| 75 | pcDNA3-myc-FSH-α (K75R) |
| 99 | pcDNA3-myc-FSH-α (K99R) |
| 115 | pcDNA3-myc-FSH-α (K115R) |
| 67 | pcDNA3-myc-FSH-β (K67R) |
| 104 | pcDNA3-myc-FSH-β (K104R) |
| 128 | pcDNA3-myc-FSH-β (K128R) |

### 2. In vivo ubiquitination assay

Plasmids encoding pcDNA3-myc-FSH-α WT and pMT123-HA-ubiquitin DNA were used to transfect HEK 293T cells. In order to identify a ubiquitination process, 3 µg of each of pcDNA3-myc-FSH-α WT and pcDNA3-myc-FSH-β WT and 1 µg of pMT123-HA-ubiquitin DNA were co-transfected into the cells; and, after 24 hours, the cells were subjected to treatment with MG132 (proteasome inhibitor, 5 µg/ml) for 6 hours, followed by immunoprecipitation assay (FIG. 28). In addition, in order to compare the level of ubiquitination between WT and the substituted ones, 3 µg of each of pcDNA3-myc-FSH-α WT, substituted pcDNA3-myc-FSH-α (K75R), substituted pcDNA3-myc-FSH-α (K99R), and substituted pcDNA3-myc-FSH-α (K115R), or 3 µg of each of substituted pcDNA3-myc-FSH-β (K67R), substituted pcDNA3-myc-FSH-β (K104R), and substituted pcDNA3-myc-FSH-β (K128R), and 1 µg of pMT123-HA-ubiquitin DNA were co-transfected into HEK 293T cells (ATCC, CRL-3216); and, after 24 hours, immunoprecipitation assay was performed (FIG. 28).

Samples obtained for immunoprecipitation were dissolved in lysis buffer (1% Triton X, 150 mM NaCl, 50 mM Tris-HCl, pH 8, and 1 mM phenylmethanesulfonyl fluoride (PMSF)), and then mixed with an anti-myc (9E10) primary antibody. Incubation was performed overnight at 4°C. Immunoprecipitates were separated by reaction at 4°C for 2 hours using Protein A/G beads (Santa Cruz Biotechnology). Then, washing with lysis buffer was performed twice. In immunoblotting, the resultant was mixed with 2X SDS buffer, and then heated at 100°C for 7 minutes, followed by SDS-PAGE to separate protein samples. The separated protein was transferred to a PVDF membrane, and then development was performed with an ECL system (Western blot detection kit, AbFrontier, Seoul, Korea) using anti-myc (9E10, Santa Cruz Biotechnology, sc-40), a blocking solution containing, at a weight ratio of 1:1,000, anti-HA (Santa Cruz Biotechnology, sc-7392) and anti-β-actin (Santa Cruz Biotechnology, sc-47778), and an anti-mouse (peroxidase-labeled antibody to mouse IgG (H+L), KPL, 074-1806) secondary antibody.

As a result, in a case where immunoprecipitation is performed with anti-myc (9E10, sc-40), as ubiquitin binds to the pcDNA3-myc-FSH-α WT and the pcDNA3-myc-FSH-β WT so that polyubiquitination is formed, a smear of ubiquitin was detected, and thus dark bands appeared (FIG. 28, lanes 3, 4, 7, and 8). In addition, in a case of being treated with MG132 (proteasome inhibitor, 5 µg/ml) for 6 hours, polyubiquitination formation increased, and thus darker bands appeared where ubiquitin is detected (FIG. 28, lanes 4 and 8). In addition, the substituted pcDNA3-myc-FSH-α (K99R), the substituted pcDNA3-myc-FSH-β (K67R), the substituted pcDNA3-myc-FSH-β (K104R), or the substituted pcDNA3-myc-FSH-β (K128R) exhibited a band lighter than WT; and the substituted pcDNA3-myc-FSH-α (K99R), the substituted pcDNA3-myc-FSH-β (K67R), the substituted pcDNA3-myc-FSH-β (K104R), or the substituted pcDNA3-myc-FSH-β (K128R) failed to bind with ubiquitin so that less ubiquitin was detected (FIG. 29, lanes 4, and 8 to 10). The above results show that FSH binds to ubiquitin and polyubiquitination occurs via the ubiquitin-proteasome system for its degradation.

### 3. Identification of half-life of FSH-α and FSH-β by protein synthesis inhibitor cycloheximide (CHX)

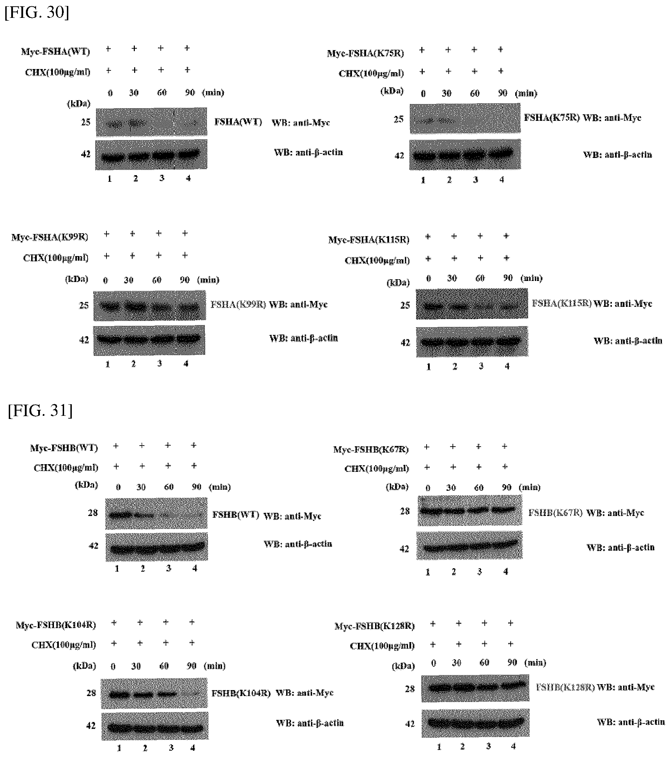

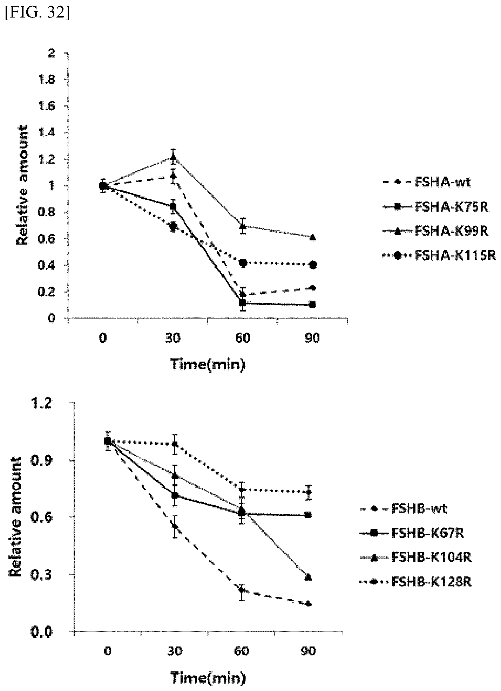

3 µg of each of pcDNA3-myc-FSH-α WT, substituted pcDNA3-myc-FSH-α (K75R), substituted pcDNA3-myc-FSH-α (K99R), and substituted pcDNA3-myc-FSH-α (K115R), or 3 µg of each of pcDNA3-myc-FSH-β WT, substituted pcDNA3-myc-FSH-β (K67R), substituted pcDNA3-myc-FSH-β (K104R), and substituted pcDNA3-myc-FSH-β (K128R) was transfected into HEK 293T cells. 48 hours after transfection, treatment with the protein synthesis inhibitor cycloheximide (CHX) (Sigma-Aldrich) (100 µg/ml) was performed; and the half-life of each of the FSH-α's and the FSH-β's was measured over 30, 60, and 90 minutes. As a result, it was identified that degradation of human FSHs is suppressed (FIG. 27). The human FSH-α had a half-life of 45 minutes, whereas the human substitutued pcDNA3-myc-FSH-α (K91R) had a half-life of equal to or longer than 90 minutes, which is longer than WT; and the human FSH-β had a half-life of shorter than 30 minutes, whereas the substitutued human pcDNA3-myc-FSH-β (K104R) had a half-life of equal to or longer than 60 minutes and the substituted pcDNA3-myc-FSH-β (K67R) or the substituted pcDNA3-myc-FSH-β (K128R) had a half-life of equal to or longer than 90 minutes, which is longer than WT. These results are graphically shown (FIGS. 30, 31, and 32).

### 4. Identification of intracellular signal transduction by FSH and substitutued FSHs

It has been reported that FSH signal transduction increases intracellular cAMP, which in turn activates PKA, thereby regulating phosphorylation of transcription factors such as CREBP, and is also associated with Erk and PI3K/AKT signal transduction (Front Endocrinol (Lausanne), 6: 142, 2015; Biochem J., 473 (11): 1483-1501, 2016).

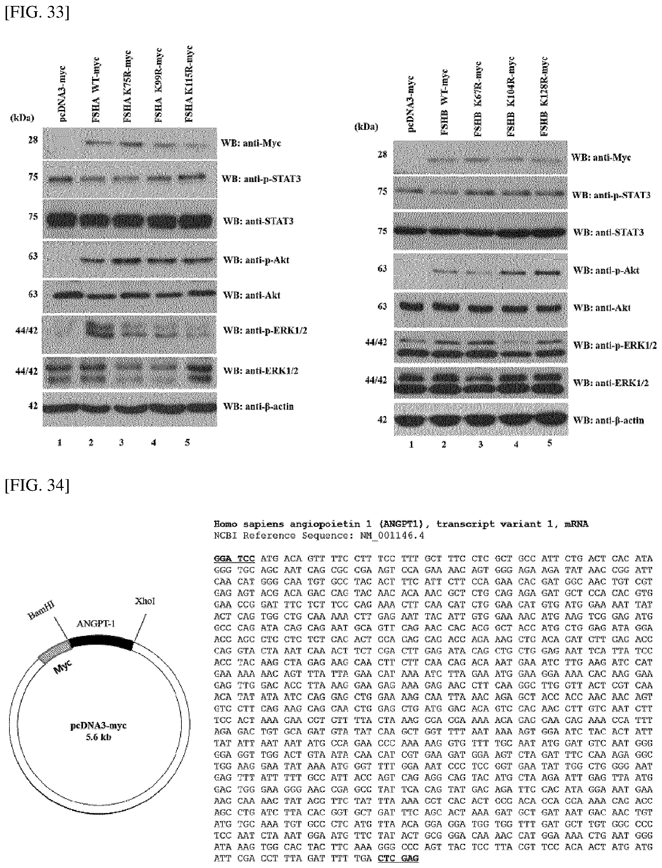

In the present example, intracellular signal transduction processes by FSH-α and substitutued FSH-α's, and FSH-β and substitutued FSH-β's were identified. 3 µg of each of pcDNA3-myc-FSH-α WT, substituted pcDNA3-myc-FSH-α (K75R), substituted pcDNA3-myc-FSH-α (K99R), and substituted pcDNA3-myc-FSH-α (K115R), or 3 µg of each of pcDNA3-myc-FSH-β WT, substituted pcDNA3-myc-FSH-β (K67R), substituted pcDNA3-myc-FSH-β (K104R), and substituted pcDNA3-myc-FSH-β (K128R) was used to transfect HeLa cells. 2 days after transfection, proteins were extracted from the cells and respectively quantified. Western blotting was performed to identify intracellular signal transduction processes. In this procedure, each protein, isolated from the HeLa cells transfected with each of the substituted pcDNA3-myc-FSH-α (K75R), the substituted pcDNA3-myc-FSH-α (K99R), and the substituted pcDNA3-myc-FSH-α (K115R), was transferred to a polyvinylidene difluoride (PVDF) membrane, and then development was performed with an ECL system (Western blot detection kit, AbFrontier, Seoul, Korea) using anti-myc (9E10, Santa Cruz Biotechnology, sc-40), anti-STAT3 (Santa Cruz Biotechnology, sc-21876), anti-phospho-STAT3 (Y705, Cell Signaling Technology 9131S), anti-AKT (H-136, Santa Cruz Biotechnology, sc-8312), anti-phospho-AKT (S473, Cell Signaling Technology 9271S), anti-Erk1/2 (9B3, AbFrontier LF-MA0134), a blocking solution containing, at a weight ratio of 1:1,000 to 1:3,000, anti-phospho-Erk1/2 (Thr202/Tyr204, AbFrontier LF-PA0090) and anti-β-actin (Santa Cruz Biotechnology, sc-47778), and anti-rabbit (goat anti-rabbit IgG-HRP, Santa Cruz Biotechnology, sc-2004) and anti-mouse (peroxidase-labeled antibody to mouse IgG (H+L), KPL, 074-1806) secondary antibodies. As a result, the substituted pcDNA3-myc-FSH-α (K75R), the substituted pcDNA3-myc-FSH-α (K99R), and the substituted pcDNA3-myc-FSH-α (K115R) exhibited, within HeLa cells, phospho-AKT signal transduction which is the same or increased as compared with the pcDNA3-myc-FSH-α WT; and the substituted pcDNA3-myc-FSH-β (K67R), the substituted pcDNA3-myc-FSH-β (K104R), and the substituted pcDNA3-myc-FSH-β (K128R) exhibited, within HeLa cells, phospho-STAT3, phospho-AKT, and phospho-Erk1/2 signal transduction which is the same or increased as compared with the pcDNA3-myc-FSH-β WT (FIG. 33).

### Example 5: Ubiquitination assay of ANGPT-1 protein, and identification of extended half-life thereof and intracellular signal transduction thereof

### 1. Cloning into expression vector and identification of protein expression

### (1) Cloning into expression vector

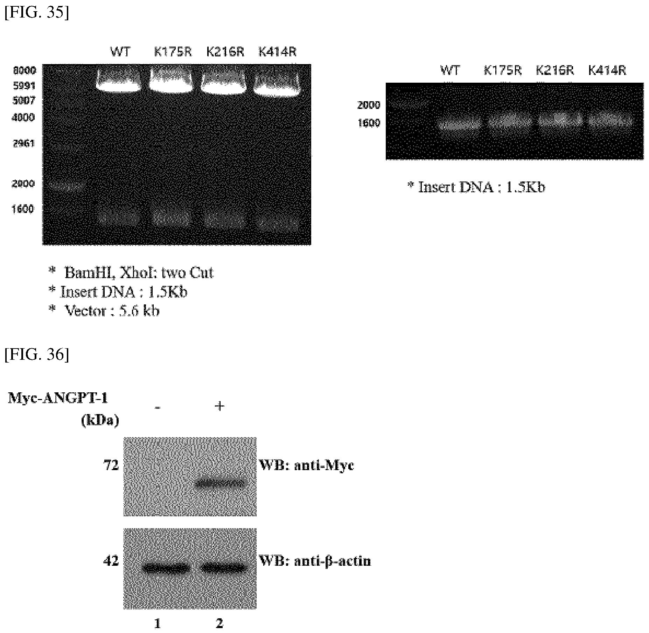

ANGPT-1 DNA amplification products obtained by polymerase chain reaction, and pcDNA3-myc (5.6 kb) were fragmented with restriction enzymes BamHI and XhoI. Then, conjugation and cloning were performed (FIG. 34, amino acid sequence of ANGPT-1: SEQ ID NO: 41). The results were identified by cleavage with restriction enzymes and then agarose gel electrolysis (FIG. 35). In addition, the underlined and bold letters on the nucleotide sequence of FIG. 34 were portions of primer sets used when polymerase chain reaction is performed to identify the cloned sites again, and the results were identified by agarose gel electrophoresis (FIG. 35). The polymerase chain reaction condition was as follows: After initial denaturation at 94°C for 3 minutes, 25 cycles of denaturation at 94°C for 30 seconds, annealing at 60°C for 30 seconds, and extension at 72°C for 1 minute and 30 seconds were used, followed by reaction at 72°C for 10 minutes. In order to identify whether the DNA thus produced is properly expressed as a protein, expression of myc present in the pcDNA3-myc vector shown in the map of FIG. 34 was checked through Western blotting using an anti-myc (9E10, Santa Cruz Biotechnology, sc-40) antibody. It was identified that the ANGPT-1 protein bound to myc is well expressed, and a blot identified as actin showed that an adequate amount of ANGPT-1 has been loaded on myc (FIG. 36).

### (2) Substitution of lysine (K) residue

Site-directed mutagenesis was used to substitute a lysine residue with arginine, and primers (ANGPT-1 K175R FP 5'-ACCTACAAGCTAGAGAGGCAACTTCTTCAA-3' (SEQ ID NO: 42), RP 5'-TTGAAGAAGTTGCCTCTCTAGCTTGTAGGT-3' (SEQ ID NO: 43), ANGPT-1 K216R FP 5'-ACCTTAAAGGAAGAGAGAGAGAACCTTCAA-3' (SEQ ID NO: 44), RP 5'-TTGAAGGTTCTCTCTCTCTTCCTTTAAGGT-3' (SEQ ID NO: 45) ANGPT-1 K414R FP 5'-GGGACAGCAGGAAGACAGAGCAGC-3' (SEQ ID NO: 46), RP 5'-GCTGCTCTGTCTTCCTGCTGTCCC-3' (SEQ ID NO: 47)) were prepared using a DNA sequence for which a specific mutation is to be induced; and then PCR was performed under a specific condition to construct plasmid DNAs in which substitution of a specific amino acid residue had occurred. Using pcDNA3-myc-ANGPT-1 as a template, plasmid DNAs, in which a lysine residue had been substituted with arginine (K → R), were constructed (Table 5).

**[Table 5]**

| Lysine (K) residue site | ANGPT-1 construct in which lysine (K) has been substituted with arginine (R) |
|---|---|
| 175 | pcDNA3-myc-ANGPT-1 (K175R) |
| 216 | pcDNA3-myc-ANGPT-1 (K216R) |
| 414 | pcDNA3-myc-ANGPT-1 (K414R) |

### 2. In vivo ubiquitination assay

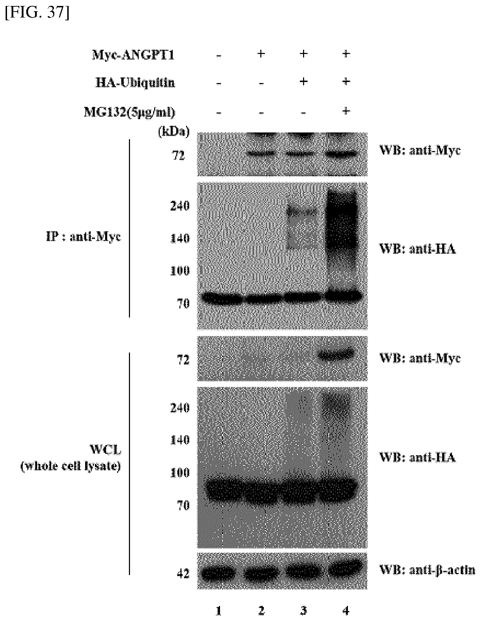

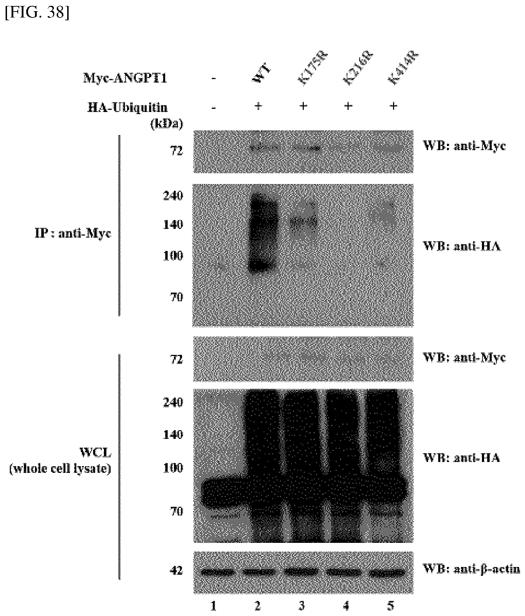

Plasmids encoding pcDNA3-myc-ANGPT-1 WT and pMT123-HA-ubiquitin DNA were used to transfect HEK 293T cells. In order to identify a ubiquitination process, 3 µg of pcDNA3-myc-ANGPT-1 WT and 1 µg of pMT123-HA-ubiquitin DNA were co-transfected into the cells; and, after 24 hours, the cells were subjected to treatment with MG132 (proteasome inhibitor, 5 µg/ml) for 6 hours, followed by immunoprecipitation assay (FIG. 37). In addition, in order to compare the level of ubiquitination between WT and the substituted ones, 3 µg of each of pcDNA3-myc-ANGPT-1 WT, substituted pcDNA3-myc-ANGPT-1 (K175R), substituted pcDNA3-myc-ANGPT-1 (K216R), and substituted pcDNA3-myc-ANGPT-1 (K414R), and 1 µg of pMT123-HA-ubiquitin DNA were co-transfected into HEK 293T cells (ATCC, CRL-3216); and, after 24 hours, immunoprecipitation assay was performed (FIG. 38).

Samples obtained for immunoprecipitation were dissolved in lysis buffer (1% Triton X, 150 mM NaCl, 50 mM Tris-HCl, pH 8, and 1 mM phenylmethanesulfonyl fluoride (PMSF)), and then mixed with an anti-myc (9E10) primary antibody. Incubation was performed overnight at 4°C. Immunoprecipitates were separated by reaction at 4°C for 2 hours using Protein A/G beads (Santa Cruz Biotechnology). Then, washing with lysis buffer was performed twice. In immunoblotting, the resultant was mixed with 2X SDS buffer, and then heated at 100°C for 7 minutes, followed by SDS-PAGE to separate protein samples. The separated protein was transferred to a PVDF membrane, and then development was performed with an ECL system (Western blot detection kit, AbFrontier, Seoul, Korea) using anti-myc (9E10, Santa Cruz Biotechnology, sc-40), a blocking solution containing, at a weight ratio of 1:1,000, anti-HA (Santa Cruz Biotechnology, sc-7392) and anti-β-actin (Santa Cruz Biotechnology, sc-47778), and an anti-mouse (peroxidase-labeled antibody to mouse IgG (H+L), KPL, 074-1806) secondary antibody.

As a result, in a case where immunoprecipitation is performed with anti-myc (9E10, Santa Cruz Biotechnology, sc-40), as ubiquitin binds to the pcDNA3-myc-ANGPT-1 WT so that polyubiquitination is formed, a smear of ubiquitin was detected, and thus dark bands appeared (FIG. 37, lanes 3 and 4). In addition, in a case of being treated with MG132 (proteasome inhibitor, 5 µg/ml) for 6 hours, polyubiquitination formation increased, and thus a darker band appeared where ubiquitin is detected (FIG. 37, lane 4). In addition, the substituted pcDNA3-myc-ANGPT-1 (K175R), the substituted pcDNA3-myc-ANGPT-1 (K216R), or the substituted pcDNA3-myc-ANGPT-1 (K414R) exhibited a band lighter than WT, and the substituted pcDNA3-myc-ANGPT-1 (K175R), substituted pcDNA3-myc-ANGPT-1 (K216R), or substituted pcDNA3-myc-ANGPT-1 (K414R) failed to bind with ubiquitin so that less ubiquitin was detected (FIG. 38, lanes 3 to 5). The above results show that ANGPT-1 binds to ubiquitin and polyubiquitination occurs via the ubiquitin-proteasome system for its degradation.

### 3. Identification of half-life of ANGPT-1 by protein synthesis inhibitor cycloheximide (CHX)

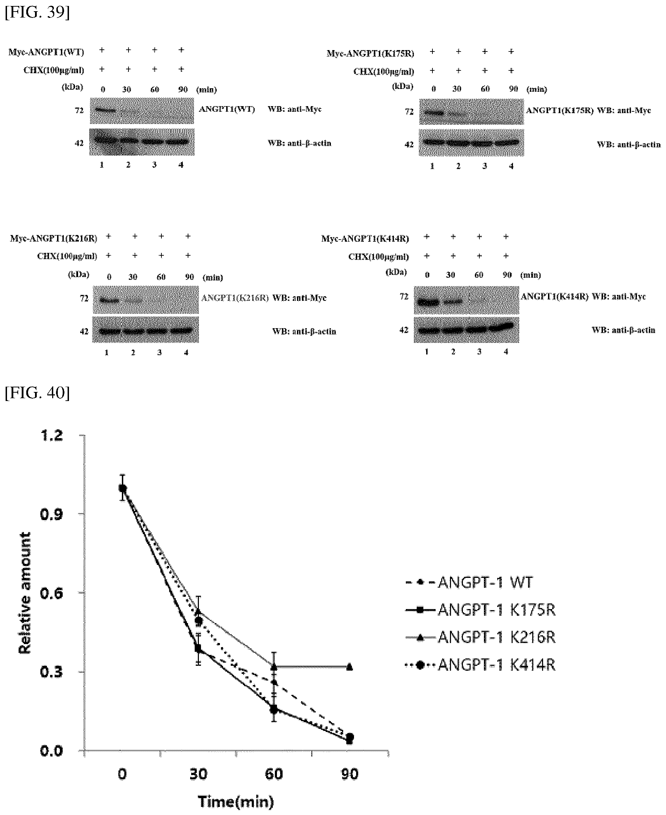

3 µg of each of pcDNA3-myc-ANGPT-1 WT, substituted pcDNA3-myc-ANGPT-1 (K175R), substituted pcDNA3-myc-ANGPT-1 (K216R), and substituted pcDNA3-myc-ANGPT-1 (K414R) was transfected into HEK 293T cells. 48 hours after transfection, treatment with the protein synthesis inhibitor cycloheximide (CHX) (Sigma-Aldrich) (100 µg/ml) was performed; and the half-life of each of the ANGPT-1's was measured over 30, 60, and 90 minutes. As a result, it was identified that degradation of human ANGPT-1's was suppressed (FIGS. 39 and 40). The human ANGPT-1 had a half-life of shorter than 20 minutes, whereas the human substituted pcDNA3-myc-ANGPT-1 (K216R) had a half-life of equal to or longer than 30 minutes, which is longer than WT. These results are graphically shown (FIGS. 39 and 40).

### 4. Identification of intracellular signal transduction by ANGPT-1 and substitutued ANGPT-1's

It has been reported that ANGPT-1 activates MAPK and PI3K/AKT signal transduction by intracellular ANGPTTIE signaling pathways, and is subsequently involved in inter-endothelial interactions and cell growth (Nat Rev Cancer, 10 (8): 575-585, 2010).

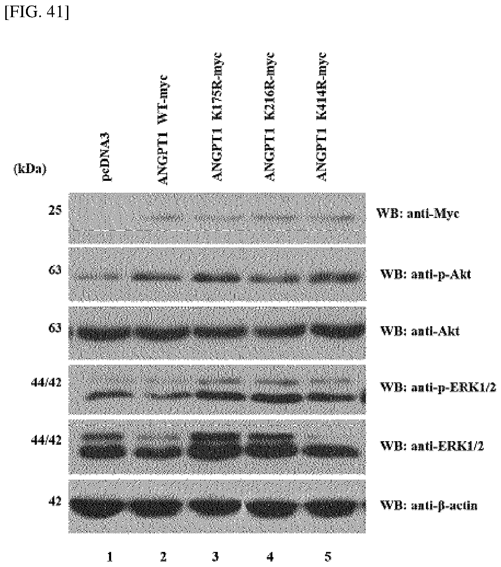

In the present example, intracellular signal transduction processes by ANGPT-1 and substitutued ANGPT-1's were identified. 5 µg of each of substituted pcDNA3-myc-ANGPT-1 (K175R), substituted pcDNA3-myc-ANGPT-1 (K216R), and substituted pcDNA3-myc-ANGPT-1 (K414R) was used to transfect HeLa cells. 2 days after transfection, proteins were extracted from the cells and respectively quantified. Western blotting was performed to identify intracellular signal transduction processes. In this procedure, each protein, isolated from the HeLa cells transfected with each of the substituted pcDNA3-myc-ANGPT-1 (K175R), the substituted pcDNA3-myc-ANGPT-1 (K216R), and the substituted pcDNA3-myc-ANGPT-1 (K414R), was transferred to a polyvinylidene difluoride (PVDF) membrane, and then development was performed with an ECL system (Western blot detection kit, AbFrontier, Seoul, Korea) using anti-myc (9E10, Santa Cruz Biotechnology, sc-40), anti-STAT3 (Santa Cruz Biotechnology, sc-21876), anti-phospho-STAT3 (Y705, Cell Signaling Technology 9131S), anti-AKT (H-136, Santa Cruz Biotechnology, sc-8312), anti-phospho-AKT (S473, Cell Signaling Technology 9271S), anti-Erk1/2 (9B3, AbFrontier LF-MA0134), a blocking solution containing, at a weight ratio of 1:1,000 to 1:3,000, anti-phospho-Erk1/2 (Thr202/Tyr204, AbFrontier LF-PA0090) and anti-β-actin (Santa Cruz Biotechnology, sc-47778), and anti-rabbit (goat anti-rabbit IgG-HRP, Santa Cruz Biotechnology, sc-2004) and anti-mouse (peroxidase-labeled antibody to mouse IgG (H+L), KPL, 074-1806) secondary antibodies. As a result, the substituted pcDNA3-myc-ANGPT-1 (K175R), the substituted pcDNA3-myc-ANGPT-1 (K216R), and the substituted pcDNA3-myc-ANGPT-1 (K414R) exhibited, within HeLa cells, phospho-AKT and phospho-Erk1/2 signal transduction which is the same or increased as compared with the pcDNA3-myc-ANGPT-1 WT (FIG. 41).

### Industrial availability

According to the present invention, there is provided a protein or (poly)peptide with an extended half-life. Therefore, the present invention relates to a protein or (poly)peptide which can be used as a therapeutic agent, and may be usefully used in the pharmaceutical industry and the cosmetic industry.

## Claims

1. A method for prolonging the half-life of a protein or (poly)peptide, comprising:
substituting at least one lysine, which binds to the C-terminal glycine of ubiquitin, with arginine in the protein or (poly)peptide.

2. The method of claim 1, wherein the protein is EGF.

3. The method of claim 2, wherein the EGF has the amino acid sequence of SEQ ID NO: 1, of which at least one of the 28^{th} and 48^{th} lysine residues from the N-terminus is substituted with arginine.

4. The method of claim 1, wherein the protein is PDGFA.

5. The method of claim 4, wherein the PDGFA has the amino acid sequence of SEQ ID NO: 6, of which at least one of the 160^{th}, 165^{th}, and 206^{th} lysine residues from the N-terminus is substituted with arginine.

6. The method of claim 1, wherein the protein is PDGFB.

7. The method of claim 6, wherein the PDGFB has the amino acid sequence of SEQ ID NO: 7, of which at least one of the 162^{nd}, 167^{th}, and 179^{th} lysine residues from the N-terminus is substituted with arginine.

8. The method of claim 1, wherein the protein is GM-CSF.

9. The method of claim 8, wherein the GM-CSF has the amino acid sequence of SEQ ID NO: 20, of which at least one of the 89^{th}, 91^{st}, and 102^{nd} lysine residues from the N-terminus is substituted with arginine.

10. The method of claim 1, wherein the protein is FSH-α.

11. The method of claim 10, wherein the FSH-α has the amino acid sequence of SEQ ID NO: 27, of which at least one of the 75^{th}, 99^{th}, and 115^{th} lysine residues from the N-terminus is substituted with arginine.

12. The method of claim 1, wherein the protein is FSH-β.

13. The method of claim 12, wherein the FSH-β has the amino acid sequence of SEQ ID NO: 28, of which at least one of the 67^{th}, 104^{th}, and 128^{th} lysine residues from the N-terminus is substituted with arginine.

14. The method of claim 1, wherein the protein is ANGPT-1.

15. The method of claim 14, wherein the ANGPT-1 has the amino acid sequence of SEQ ID NO: 41, of which at least one of the 175^{th}, 216^{th}, and 414^{th} lysine residues from the N-terminus is substituted with arginine.

16. A protein with an extended half-life, wherein at least one of the lysine residues of the protein is substituted with arginine, and the lysine residue to be substituted binds to the C-terminal glycine of ubiquitin.

17. The protein of claim 16, wherein the protein with an extended half-life is selected from growth hormone releasing hormone (GHRH), growth hormone releasing peptide, interferons (interferon-a or interferon-β), interferon receptors, colony stimulating factors (CSFs), glucagon-like peptides, interleukins, interleukin receptors, enzymes, interleukin binding proteins, cytokine binding proteins, G-protein-coupled receptors, human growth hormone (hGH), macrophage activating factor, macrophage peptide, B cell factor, T cell factor, protein A, allergy inhibitors, cell necrosis glycoproteins, G-protein-coupled receptors, immunotoxins, lymphotoxins, tumor necrosis factor, tumor suppressors, metastasis growth factor, alpha-1 antitrypsin, albumin, alpha-lactalbumin, apolipoprotein-E, erythropoietin, highly glycosylated erythropoietin, angiopoietins, hemoglobin, thrombin, thrombin receptor activating peptide, thrombomodulin, factor VII, factor VIIa, factor VIII, factor IX, factor XIII, plasminogen activating factor, urokinase, streptokinase, hirudin, protein C, C-reactive protein, renin inhibitors, collagenase inhibitors, superoxide dismutase, leptin, platelet-derived growth factor, epithelial growth factor, epidermal growth factor, angiostatin, angiotensin, bone growth factor, bone stimulating protein, calcitonin, insulin, atriopeptin, cartilage inducing factor, fibrin-binding peptide, elcatonin, connective tissue activating factor, tissue factor pathway inhibitors, follicle stimulating hormone, luteinizing hormone, luteinizing hormone releasing hormone, nerve growth factors, parathyroid hormone, relaxin, secretin, somatomedin, insulin-like growth factor, adrenocortical hormone, glucagon, cholecystokinin, pancreatic polypeptide, gastrin releasing peptide, corticotropin releasing factor, thyroid stimulating hormone, autotaxin, lactoferrin, myostatin, receptors, receptor antagonists, cell surface antigens, virus derived vaccine antigens, monoclonal antibodies, polyclonal antibodies, or antibody fragments.

18. The protein of claim 16, wherein the protein is EGF having SEQ ID NO: 1, of which at least one of the 28^{th} and 48^{th} lysine residues from the N-terminus is substituted with arginine.

19. The protein of claim 16, wherein the protein is PDGFA having SEQ ID NO: 6, of which at least one of the 160^{th}, 165^{th}, and 206^{th} lysine residues from the N-terminus is substituted with arginine.

20. The protein of claim 16, wherein the protein is PDGFB having SEQ ID NO: 7, of which at least one of the 162^{nd}, 167^{th}, and 179^{th} lysine residues from the N-terminus is substituted with arginine.

21. The protein of claim 16, wherein the protein is GM-CSF having SEQ ID NO: 20, of which at least one of the 89^{th}, 91^{st}, and 102^{nd} lysine residues from the N-terminus is substituted with arginine.

22. The protein of claim 16, wherein the protein is FSH-α having SEQ ID NO: 27, of which at least one of the 75^{th}, 99^{th}, and 115^{th} lysine residues from the N-terminus is substituted with arginine.

23. The protein of claim 16, wherein the protein is FSH-β having SEQ ID NO: 28, of which at least one of the 67^{th}, 104^{th}, and 128^{th} lysine residues from the N-terminus is substituted with arginine.

24. The protein of claim 16, wherein the protein is ANGPT-1 having SEQ ID NO: 41, of which at least one of the 175^{th}, 216^{th}, and 414^{th} lysine residues from the N-terminus is substituted with arginine.

25. A pharmaceutical and/or cosmetic composition for cell growth, skin and hair regeneration, and/or treatment, comprising:
the EGF of claim 18; and
an excipient.

26. A pharmaceutical and/or cosmetic composition for cell growth, angiogenesis, and recovery from chronic ulcers and bone loss, comprising:
the PDGFA of claim 19 or the PDGFB of claim 20; and
an excipient

27. A pharmaceutical composition for prevention of neutropenia and/or prevention and/or treatment of an immune disease and/or cancer including solid cancer and hematological cancer and/or rheumatoid arthritis, comprising:
the GM-CSF of claim 21; and
a pharmaceutically acceptable excipient.

28. An infertility therapeutic agent for inducing superovulation and/or pharmaceutical composition for treatment of solid cancer, comprising:
the FSH-α of claim 22 or the FSH-β of claim 23; and
a pharmaceutically acceptable excipient.

29. A pharmaceutical composition for treatment of diabetes, a cardiac disease, and/or sepsis, comprising:
the ANGPT-1 of claim 24; and
a pharmaceutically acceptable excipient.

30. An expression vector, comprising:
(a) a promoter;
(b) a nucleotide sequence encoding the protein of any one of claims 16 to 24; and any linker,
wherein the promoter and the nucleotide sequence are operably linked to each other.

31. A host cell, comprising:
the expression vector of claim 30.


<110>     UbiProtein. Corp

<120>     A method for extending half-life of a protein

<130>     UBPRN18P01WO

<160>     47

<170>     KoPatentIn 3.0

<210>     1
<211>     53
<212>     PRT
<213>     Artificial Sequence

<220>
<223>     EGF


<400>     1
Asn Ser Asp Ser Glu Cys Pro Leu Ser His Asp Gly Tyr Cys Leu His
  1               5                  10                  15

Asp Gly Val Cys Met Tyr Ile Glu Ala Leu Asp Lys Tyr Ala Cys Asn
             20                  25                  30

Cys Val Val Gly Tyr Ile Gly Glu Arg Cys Gln Tyr Arg Asp Leu Lys
         35                  40                  45

Trp Trp Glu Leu Arg
     50


<210>     2
<211>     27
<212>     DNA
<213>     Artificial Sequence

<220>
<223>     Primer


<400>     2
gaagcattgg acaggtatgc atgcaac                                          27


<210>     3
<211>     27
<212>     DNA
<213>     Artificial Sequence

<220>
<223>     Primer


<400>     3
gttgcatgca tacctgtcca atgcttc                                          27


<210>     4
<211>     27
<212>     DNA
<213>     Artificial Sequence

<220>
<223>    Primer


<400>    4
taccgagacc tgaggtggtg ggaactg                                          27


<210>    5
<211>    27
<212>    DNA
<213>    Artificial Sequence

<220>
<223>    Primer


<400>    5
cagttcccac cacctcaggt ctcggta                                          27


<210>    6
<211>    211
<212>    PRT
<213>    Artificial Sequence

<220>
<223>    PDGFA


<400>    6



<210>    7
<211>    241
<212>    PRT
<213>    Artificial Sequence

<220>
<223>    PDGFB


<400>    7

<210>    8
<211>    27
<212>    DNA
<213>    Artificial Sequence

<220>
<223>    Primer

<400>    8
gaatacgtca ggaggaagcc aaaatta                                          27

<210>    9
<211>    27
<212>    DNA
<213>    Artificial Sequence

<220>
<223>    Primer

<400>    9
taattttggc ttcctcctga cgtattc                                          27

<210>    10
<211>    27
<212>    DNA
<213>    Artificial Sequence

<220>
<223>    Primer

<400>    10
aagccaaaat taagagaagt ccaggtg                                          27

<210>    11
<211>    27
<212>    DNA
<213>    Artificial Sequence

<220>
<223>    Primer

<400>    11
cacctggact tctcttaatt ttggctt                                          27

<210>    12
<211>    27
<212>    DNA

<213>    Artificial Sequence

<220>
<223>    Primer


<400>    12
aaacggaaaa gaagaaggtt aaaaccc                                          27


<210>    13
<211>    27
<212>    DNA
<213>    Artificial Sequence

<220>
<223>    Primer


<400>    13
gggttttaac cttcttcttt tccgttt                                          27


<210>    14
<211>    24
<212>    DNA
<213>    Artificial Sequence

<220>
<223>    Primer


<400>    14
attgtgcgga agaggccaat cttt                                             24


<210>    15
<211>    24
<212>    DNA
<213>    Artificial Sequence

<220>
<223>    Primer


<400>    15
aaagattggc ctcttccgca caat                                             24


<210>    16
<211>    24
<212>    DNA
<213>    Artificial Sequence

<220>
<223>    Primer


<400>    16
ccaatcttta agagggccac ggtg                                             24


<210>    17

<211>    24
<212>    DNA
<213>    Artificial Sequence

<220>
<223>    Primer


<400>    17
caccgtggcc ctcttaaaga ttgg                                              24


<210>    18
<211>    24
<212>    DNA
<213>    Artificial Sequence

<220>
<223>    Primer


<400>    18
cacctggcat gcaggtgtga gaca                                              24


<210>    19
<211>    24
<212>    DNA
<213>    Artificial Sequence

<220>
<223>    Primer


<400>    19
tgtctcacac ctgcatgcca ggtg                                              24


<210>    20
<211>    144
<212>    PRT
<213>    Artificial Sequence

<220>
<223>    GM-CSF


<400>    20
Met Trp Leu Gln Ser Leu Leu Leu Leu Gly Thr Val Ala Cys Ser Ile
1               5                   10                  15

Ser Ala Pro Ala Arg Ser Pro Ser Pro Ser Thr Gln Pro Trp Glu His
            20                  25                  30

Val Asn Ala Ile Gln Glu Ala Arg Arg Leu Leu Asn Leu Ser Arg Asp
        35                  40                  45

Thr Ala Ala Glu Met Asn Glu Thr Val Glu Val Ile Ser Glu Met Phe
    50                  55                  60

Asp Leu Gln Glu Pro Thr Cys Leu Gln Thr Arg Leu Glu Leu Tyr Lys
65                  70                  75                  80

Gln Gly Leu Arg Gly Ser Leu Thr Lys Leu Lys Gly Pro Leu Thr Met
                85                  90                  95

Met Ala Ser His Tyr Lys Gln His Cys Pro Pro Thr Pro Glu Thr Ser
            100                 105                 110

Cys Ala Thr Gln Ile Ile Thr Phe Glu Ser Phe Lys Glu Asn Leu Lys
        115                 120                 125

Asp Phe Leu Leu Val Ile Pro Phe Asp Cys Trp Glu Pro Val Gln Glu
    130                 135                 140

<210>    21
<211>    25
<212>    DNA
<213>    Artificial Sequence

<220>
<223>    Primer

<400>    21
ggcagcctca ccaggctcaa gggcc                                          25

<210>    22
<211>    25
<212>    DNA
<213>    Artificial Sequence

<220>
<223>    Primer

<400>    22
ggcccttgag cctggtgagg ctgcc                                          25

<210>    23
<211>    27
<212>    DNA
<213>    Artificial Sequence

<220>
<223>    Primer

<400>    23
ctcaccaagc tcagggcccc cttgacc                                        27

<210>    24
<211>    27
<212>    DNA
<213>    Artificial Sequence

<220>
<223>    Primer

<400>    24
ggtcaagggg cccctgagct tggtgag                                          27


<210>    25
<211>    27
<212>    DNA
<213>    Artificial Sequence

<220>
<223>    Primer


<400>    25
gctagccact acagacagca ctgccct                                          27


<210>    26
<211>    27
<212>    DNA
<213>    Artificial Sequence

<220>
<223>    Primer


<400>    26
agggcagtgc tgtctgtagt ggctagc                                          27


<210>    27
<211>    116
<212>    PRT
<213>    Artificial Sequence

<220>
<223>    FSH Alpha


<400>    27
Met Asp Tyr Tyr Arg Lys Tyr Ala Ala Ile Phe Leu Val Thr Leu Ser
1               5                   10                  15

Val Phe Leu His Val Leu His Ser Ala Pro Asp Val Gln Asp Cys Pro
            20                  25                  30

Glu Cys Thr Leu Gln Glu Asn Pro Phe Phe Ser Gln Pro Gly Ala Pro
        35                  40                  45

Ile Leu Gln Cys Met Gly Cys Cys Phe Ser Arg Ala Tyr Pro Thr Pro
    50                  55                  60

Leu Arg Ser Lys Lys Thr Met Leu Val Gln Lys Asn Val Thr Ser Glu
65                  70                  75                  80

Ser Thr Cys Cys Val Ala Lys Ser Tyr Asn Arg Val Thr Val Met Gly
                85                  90                  95

Gly Phe Lys Val Glu Asn His Thr Ala Cys His Cys Ser Thr Cys Tyr
            100                 105                 110

Tyr His Lys Ser
        115

<210>    28
<211>    129
<212>    PRT
<213>    Artificial Sequence

<220>
<223>    FSH Beta

<400>    28
Met Lys Thr Leu Gln Phe Phe Phe Leu Phe Cys Cys Trp Lys Ala Ile
1               5                   10                  15

Cys Cys Asn Ser Cys Glu Leu Thr Asn Ile Thr Ile Ala Ile Glu Lys
            20                  25                  30

Glu Glu Cys Arg Phe Cys Ile Ser Ile Asn Thr Thr Trp Cys Ala Gly
        35                  40                  45

Tyr Cys Tyr Thr Arg Asp Leu Val Tyr Lys Asp Pro Ala Arg Pro Lys
    50                  55                  60

Ile Gln Lys Thr Cys Thr Phe Lys Glu Leu Val Tyr Glu Thr Val Arg
65                  70                  75                  80

Val Pro Gly Cys Ala His His Ala Asp Ser Leu Tyr Thr Tyr Pro Val
                85                  90                  95

Ala Thr Gln Cys His Cys Gly Lys Cys Asp Ser Asp Ser Thr Asp Cys
            100                 105                 110

Thr Val Arg Gly Leu Gly Pro Ser Tyr Cys Ser Phe Gly Glu Met Lys
        115                 120                 125

Glu

<210>    29
<211>    24
<212>    DNA
<213>    Artificial Sequence

<220>
<223>    Primer

<400>    29
atgttggtcc aaaggaacgt cacc                                              24

<210>    30
<211>    24
<212>    DNA
<213>    Artificial Sequence

<220>
<223>    Primer

<400>    30

ggtgacgttc ctttggacca acat                                          24

<210>    31
<211>    24
<212>    DNA
<213>    Artificial Sequence

<220>
<223>    Primer

<400>    31
   atgggggttt tcagagtgga gaac                                          24

<210>    32
<211>    24
<212>    DNA
<213>    Artificial Sequence

<220>
<223>    Primer

<400>    32
gttctccact ctgaaacccc ccat                                          24

<210>    33
<211>    27
<212>    DNA
<213>    Artificial Sequence

<220>
<223>    Primer

<400>    33
tgttattatc acagatctta actcgag                                       27

<210>    34
<211>    27
<212>    DNA
<213>    Artificial Sequence

<220>
<223>    Primer

<400>    34
ctcgagttaa gatctgtgat aataaca                                       27

<210>    35
<211>    28
<212>    DNA
<213>    Artificial Sequence

<220>
<223>    Primer

<400>    35
ggcccaaaat ccagagaaca tgtacctt                                          28

<210>    36
<211>    28
<212>    DNA
<213>    Artificial Sequence

<220>
<223>    Primer

<400>    36
aaggtacatg ttctctggat tttgggcc                                          28

<210>    37
<211>    25
<212>    DNA
<213>    Artificial Sequence

<220>
<223>    Primer

<400>    37
gtcactgtgg caggtgtgac agcga                                             25

<210>    38
<211>    25
<212>    DNA
<213>    Artificial Sequence

<220>
<223>    Primer

<400>    38
tcgctgtcac acctgccaca gtgac                                             25

<210>    39
<211>    27
<212>    DNA
<213>    Artificial Sequence

<220>
<223>    Primer

<400>    39
ggtgaaatga gagaaacgcg tacgcgg                                           27

<210>    40
<211>    27
<212>    DNA
<213>    Artificial Sequence

<220>

<223>    Primer

<400>    40
ccgcgtacgc gtttctctca tttcacc                                          27

<210>    41
<211>    498
<212>    PRT
<213>    Artificial Sequence

<220>
<223>    Angiopoietin-1

<400>    41
Met Thr Val Phe Leu Ser Phe Ala Phe Leu Ala Ala Ile Leu Thr His
1               5                   10                  15

Ile Gly Cys Ser Asn Gln Arg Arg Ser Pro Glu Asn Ser Gly Arg Arg
            20                  25                  30

Tyr Asn Arg Ile Gln His Gly Gln Cys Ala Tyr Thr Phe Ile Leu Pro
        35                  40                  45

Glu His Asp Gly Asn Cys Arg Glu Ser Thr Thr Asp Gln Tyr Asn Thr
    50                  55                  60

Asn Ala Leu Gln Arg Asp Ala Pro His Val Glu Pro Asp Phe Ser Ser
65                  70                  75                  80

Gln Lys Leu Gln His Leu Glu His Val Met Glu Asn Tyr Thr Gln Trp
                85                  90                  95

Leu Gln Lys Leu Glu Asn Tyr Ile Val Glu Asn Met Lys Ser Glu Met
            100                 105                 110

Ala Gln Ile Gln Gln Asn Ala Val Gln Asn His Thr Ala Thr Met Leu
        115                 120                 125

Glu Ile Gly Thr Ser Leu Leu Ser Gln Thr Ala Glu Gln Thr Arg Lys
    130                 135                 140

Leu Thr Asp Val Glu Thr Gln Val Leu Asn Gln Thr Ser Arg Leu Glu
145                 150                 155                 160

Ile Gln Leu Leu Glu Asn Ser Leu Ser Thr Tyr Lys Leu Glu Lys Gln
                165                 170                 175

Leu Leu Gln Gln Thr Asn Glu Ile Leu Lys Ile His Glu Lys Asn Ser
            180                 185                 190

Leu Leu Glu His Lys Ile Leu Glu Met Glu Gly Lys His Lys Glu Glu
        195                 200                 205

Leu Asp Thr Leu Lys Glu Glu Lys Glu Asn Leu Gln Gly Leu Val Thr
    210                 215                 220

Arg Gln Thr Tyr Ile Ile Gln Glu Leu Glu Lys Gln Leu Asn Arg Ala
225                 230                 235                 240

Thr Thr Asn Asn Ser Val Leu Gln Lys Gln Gln Leu Glu Leu Met Asp

245                 250                 255

Thr Val His Asn Leu Val Asn Leu Cys Thr Lys Glu Gly Val Leu Leu
            260                 265                 270

Lys Gly Gly Lys Arg Glu Glu Glu Lys Pro Phe Arg Asp Cys Ala Asp
        275                 280                 285

Val Tyr Gln Ala Gly Phe Asn Lys Ser Gly Ile Tyr Thr Ile Tyr Ile
    290                 295                 300

Asn Asn Met Pro Glu Pro Lys Lys Val Phe Cys Asn Met Asp Val Asn
305                 310                 315                 320

Gly Gly Gly Trp Thr Val Ile Gln His Arg Glu Asp Gly Ser Leu Asp
                325                 330                 335

Phe Gln Arg Gly Trp Lys Glu Tyr Lys Met Gly Phe Gly Asn Pro Ser
            340                 345                 350

Gly Glu Tyr Trp Leu Gly Asn Glu Phe Ile Phe Ala Ile Thr Ser Gln
        355                 360                 365

Arg Gln Tyr Met Leu Arg Ile Glu Leu Met Asp Trp Glu Gly Asn Arg
    370                 375                 380

Ala Tyr Ser Gln Tyr Asp Arg Phe His Ile Gly Asn Glu Lys Gln Asn
385                 390                 395                 400

Tyr Arg Leu Tyr Leu Lys Gly His Thr Gly Thr Ala Gly Lys Gln Ser
                405                 410                 415

Ser Leu Ile Leu His Gly Ala Asp Phe Ser Thr Lys Asp Ala Asp Asn
            420                 425                 430

Asp Asn Cys Met Cys Lys Cys Ala Leu Met Leu Thr Gly Gly Trp Trp
        435                 440                 445

Phe Asp Ala Cys Gly Pro Ser Asn Leu Asn Gly Met Phe Tyr Thr Ala
    450                 455                 460

Gly Gln Asn His Gly Lys Leu Asn Gly Ile Lys Trp His Tyr Phe Lys
465                 470                 475                 480

Gly Pro Ser Tyr Ser Leu Arg Ser Thr Thr Met Met Ile Arg Pro Leu
                485                 490                 495

Asp Phe

<210>    42
<211>    30
<212>    DNA
<213>    Artificial Sequence

<220>
<223>    Primer

<400>    42
acctacaagc tagagaggca acttcttcaa                                        30

<210>    43
<211>    30
<212>    DNA
<213>    Artificial Sequence

<220>
<223>    Primer


<400>    43
ttgaagaagt tgcctctcta gcttgtaggt                                        30


<210>    44
<211>    30
<212>    DNA
<213>    Artificial Sequence

<220>
<223>    Primer


<400>    44
accttaaagg aagagagaga gaaccttcaa                                        30


<210>    45
<211>    30
<212>    DNA
<213>    Artificial Sequence

<220>
<223>    Primer


<400>    45
ttgaaggttc tctctctctt cctttaaggt                                        30


<210>    46
<211>    24
<212>    DNA
<213>    Artificial Sequence

<220>
<223>    Primer


<400>    46
gggacagcag gaagacagag cagc                                              24


<210>    47
<211>    24
<212>    DNA
<213>    Artificial Sequence

<220>
<223>    Primer


<400>    47

gctgctctgt cttcctgctg tccc 24